# EUROPÄISCHE PATENTSCHRIFT

(11) **EP 0 416 196 B1**
(45) Veröffentlichungstag und Bekanntmachung des Hinweises auf die Patenterteilung: **23.02.1994**
(21) Anmeldenummer: 90102823.3
(22) Anmeldetag: 13.02.1990
(51) Int. Cl.: H02G 11/02, B65H 75/44, A61B 6/00

(54) **Wickelvorrichtung für eine Leitungsanordnung**
Winding device for a cable arrangement
Dispositif d'enroulement pour un arrangement de câble

(30) Priorität: 31.08.1989 EP 89116119
(43) Veröffentlichungstag der Anmeldung: 13.03.1991
(73) Patentinhaber: W.L. Gore & Associates GmbH, 85636 Putzbrunn (DE)
(72) Erfinder: Haiduk, Herbert, D-8835 Pleinfeld (DE)
(74) Vertreter: Klunker . Schmitt-Nilson . Hirsch

(56) Entgegenhaltungen:
- DE-A- 3 406 221
- FR-A- 937 228
- FR-A- 2 041 352
- US-A- 4 163 526

## Beschreibung

Die Erfindung betrifft eine Wickelvorrichtung zum Auf- und Abwickeln einer Leitungsanordnung, die einen Endes mit einer feststehenden Einrichtung und anderen Endes mit einer hin- und herbeweglichen Einrichtung koppelbar ist, mit einer ortsfesten hohlzylindrischen Innentrommel und einer um diese herum drehbaren hohlzylindrischen Außentrommel, wobei die Innentrommel und die Außentrommel je mit einer Leitungsdurchführöffnung zum Hindurchführen und dort Fixieren der Leitungsanordnung versehen sind und wobei durch Drehen der Außentrommel ein außerhalb der Außentrommel befindlicher Außenbereich der Leitungsanordnung auf deren Außenumfang auf- bzw. abwickelbar und ein zwischen Außentrommel und Innentrommel befindlicher Innenbereich der Leitungsanordnung bezüglich der Innentrommel ab- bzw. aufwickelbar ist.

Es gibt Geräte, die während ihres Betriebes bewegt und in allen Bewegungsstellungen an eine mit ihnen mitbewegliche Leitungsanordnung angeschlossen sein müssen. Dabei kann es sich um elektrische oder optische Signalabgabe- und/oder Versorgungs-Leitungsanordnungen und/oder um Fluid-Leitungen, beispielsweise zur Gas- und/oder Flüssigkeitsversorgung des Gerätes handeln. Solche Geräte können auf linearen oder nicht-linearen Bahnen beweglich sein. Beispiele sind Roboter, die sich um eine Drehachse drehen und/oder eine Lateralbewegung ausführen, Meßköpfe zur Überwachung von Materialbahnen, wie Stoff- oder Papierbahnen, die zur Überwachung der gesamten Bahnbreite über die Bahn hin- und herbewegt werden, oder Röntgengeräte, die an einem etwa halbkreisförmigen C-Bogen einen Endes eine Röntgenstrahlenquelle und anderen Endes einen Röntgenstrahlenempfänger aufweisen, wobei der C-Bogen um seine Kreisbahn verschwenkbar ist, um die Röntgenstrahlenquelle und den Röntgenstrahlenempfänger immer auf die jeweilige Position, beispielsweise eines zu operierenden Patienten, einstellen zu können. Dabei wird der C-Bogen von einer Bedienungsperson, beispielsweise dem operierenden Arzt von Hand in die jeweils gewünschte Kreisbogenstellung geschwenkt, in welcher er dann exakt stehenbleiben muß.

Allen solchen Geräten ist gemeinsam, daß der bewegliche Geräteteil in jeder seiner möglichen Positionen mit einer feststehenden Einrichtung verbunden werden muß, um den beweglichen Geräteteil zu versorgen und/oder von dem beweglichen Geräteteil beispielsweise Signale abzunehmen.

Bei einem Röntgengerät der angegebenen Art, hat man sich mit einer Kabelschlaufe geholfen, die zwischen beweglichem Geräteteil und feststehendem Geräteteil angeordnet war. Dies ist aber insbesondere in einem Operationssaal problematisch, weil eine solche Kabelschlaufe nicht nur eine Stolperquelle darstellen kann sondern auch häufig im Weg ist, den Patienten belästigen kann und einen Schmutzfänger bilden kann.

Eine Vorrichtung zum Auf- und Abrollen eines elektrischen Leiters mit federunterstützter Drehtrommel für die Aufnahme des Leiters, wie sie aus der DE-34 06 221 A1 bekannt ist, hat sich zwar für viele Anwendungen bewährt. Verwendet man sie allerdings für ein Röntgengerät der oben angegeben Art, tritt das Problem auf, daß die in der Drehtrommel untergebrachte Feder über die Drehtrommel eine Rückstellkraft auf den C-Bogen des Röntgengerätes ausübt, wodurch der C-Bogen und damit die an seinem einen Ende angebrachte Röntgenstrahlenquelle und der an seinem anderen Ende angebrachte Röntgenstrahlenempfänger nicht genügend sicher in der von der Bedienungsperson eingestellten Drehstellung verbleiben.

Um dieses Problem zu überwinden, ist vorgeschlagen worden, die Drehtrommel der Vorrichtung zum Auf- und Abwickeln eines elektrischen Kabels durch das Aufwickeln bzw. Abwickeln des Kabels zu drehen. Dabei wird ein Flachkabel verwendet, dessen Steifigkeit genügend groß ist, um die Drehtrommel mit dem zur Drehtrommel hin bewegten Kabel zu drehen. Um zu erreichen, daß das Kabel beim Aufwickelvorgang enganliegend auf den Außenumfang der Drehtrommel aufgewickelt wird und es vor der Drehtrommel zu keinen schlaufenartigen Verwerfungen des zur Drehtrommel geschobenen Kabels kommt, müssen Andruckrollen vorgesehen werden, von denen eine an den Außenumfang der Drehtrommel drückt, um ein strammes Aufwickeln des Kabels auf die Drehtrommel zu erreichen, und die andere im Bereich vor der Drehtrommel gegen den C-Bogen drückt, um das Kabel auf dem C-Bogen zu halten und Verwerfungen des Kabels zu vermeiden.

Um eine saubere Kabelführung zu erreichen, sind ziemlich starke Andruckkräfte zwischen den Druckrollen und dem Kabel erforderlich. Es hat sich nun herausgestellt, daß die dadurch auf das Kabel ausgeübte Walk- und Schubwirkung von vielen Kabeln nicht verkraftet wird und es bei solchen Kabeln zu frühzeitiger Beschädigung kommt. Dies kann soweit gehen, daß sich einzelne Leiter des Kabels durch den äußeren Kabelmantel hindurch arbeiten und schlaufenartig aus dem Kabelmantel herausstehen. Mit dieser Auf- und Abwickelvorrichtung kann daher die erforderliche Standzeit bei solchen Kabeln nicht sichergestellt werden.

Der Erfindung liegt die Aufgabe zugrunde, eine Wickelvorrichtung der eingangs angegebenen Art verfügbar zu machen, welche einerseits eine genaue Positionierung der hin- und herbeweglichen Einrichtung nicht beeinträchtigt und die andererseits weder ein Hindernis für benutzende Personen noch eine übermäßige Belastung für die Leitungsanordnung darstellt.

Eine Lösung dieser Aufgabe besteht in einer Wickelvorrichtung der eingangs angegebenen Art, die dadurch gekennzeichnet ist, daß eine durch die Bewegung der hin- und herbeweglichen Einrichtung getriebene Antriebseinrichtung über eine Rutschkupplung mit der Außentrommel gekoppelt und im Verhältnis zum Außentrommeldurchmesser derart dimensioniert ist, daß die Antriebseinrichtung die Außentrommel bei rutschfreier Kupplung auf eine Umfangsgeschwindigkeit brächte, die größer wäre als die für das Wickeln des Außenbereichs der Leitungsanordnung erforderliche Umfangsgeschwindigkeit der Außentrommel, und daß die Außentrommel unter der Bremswirkung einer Bremseinrichtung steht, deren Bremskraft so dimensioniert ist, daß sie die Außentrommel in Abwickeldrehrichtung auf eine Umfangsgeschwindigkeit abzubremsen versucht, die niedriger ist als die der Abwickelgeschwindigkeit des Außenbereichs der Leitungsanordnung entsprechende Umfangsgeschwindigkeit der Außentrommel.

Da bei der erfindungsgemäßen Wickelvorrichtung die Außentrommel durch die Bewegung der hin- und herbeweglichen Einrichtung angetrieben wird, ist weder eine Rückstellfeder noch ein Anschieben der Außentrommel über die Leitungsanordnung mit den je geschilderten Problemen erforderlich. Dadurch, daß einerseits die Außentrommel so angetrieben wird, daß sie sich bei freier Beweglichkeit schneller drehen würde, als es zum Aufwickeln der Leitungsanordnung erforderlich ist, und daß andererseits die Außentrommel mit der Antriebseinrichtung über eine Rutschkupplung verbunden ist, wird ein strammes Aufwickeln der Leitungsanordnung auf dem Außenmantel der Außentrommel sichergestellt, ohne die Leitungsanordnung besonders zu belasten. Da die Außentrommel bei rutschfreier Kupplung mit der Antriebseinrichtung schneller gedreht würde, als für das Auf- und Abwickeln erforderlich, könnte sich die Außentrommel beim Abwickelvorgang, bei dem kein aufzuwickelndes Kabel bremst, zu schnell drehen, was zu einem Abheben der Leitungsanordnung von dem Außenmantel der Außentrommel und möglicherweise zu einem Verklemmen der Leitungsanordnung zwischen der Außentrommel und einem die Außentrommel umgebenden Gehäuse führen könnte. Dem wirkt die erfindungsgemäße Bremseinrichtung entgegen. Diese bremst die Außentrommel beim Abwickelvorgang soweit ab, daß sie dank der Rutschkupplung zwischen Außentrommel und Antriebseinrichtung eine solche Drehgeschwindigkeit einnimmt, wie sie durch das Wegziehen der Leitungsanordnung beim Abwickelvorgang vorgegeben wird.

Gemäß einer vorteilhaften Weiterbildung der Erfindung ist die Bremsstärke der Bremseinrichtung so einstellbar, daß einerseits durch den Bremsvorgang nur eine geringfügige Zugbelastung auf die abzuwickelnde Leitungsanordnung ausgeübt wird und andererseits die Außentrommel nur so schnell dreht, daß die Leitungsanordnung beim Abwickelvorgang stramm auf dem Außenmantel der Außentrommel gehalten wird.

Bei einer besonders bevorzugten Ausführungsform der Erfindung ist die Antriebseinrichtung durch eine Treibradeinrichtung, vorzugsweise auf beiden Stirnseiten der Außentrommel, gebildet, die durch Abrollen auf einer neben bzw. beidseits der Leitungsanordnungsbahn befindlichen Abrollbahn angetrieben wird. Bevorzugtermaßen weist die Treibradeinrichtung mindestens einen Treibradring auf, dessen Außenumfang auf der zugehörigen Abrollbahn abrollt und dessen Innenumfang den Außenumfang einer an der zugehörigen Stirnseite der Außentrommel angeordneten Außentrommelscheibe in rutschkupplungsartigem Eingriff umschließt. Dabei ist der Außendurchmesser des Treibradringes kleiner als der Außendurchmesser der Außentrommel.

In bevorzugter Ausführungsweise der Erfindung besteht der Treibradring aus elastischem Material, ist mit mindestens einem radialen Spalt versehen und wird von einem Laufring aus elastischem Material umfaßt. Dadurch wird die Rutschkupplungskraft durch die Reibungskoeffizienten der an der Rutschkupplung beteiligten Materialien und durch die vom elastischen Laufring auf den Treibradring ausgeübte Vorspannung bestimmt.

Zur Erhöhung der Reibkraft der Rutschkupplung sind die an dem Kupplungsvorgang beteiligten Flächen von Laufradring und Außentrommelscheibe dadurch vergrößert, daß man sie in komplementärer Weise ausbaucht bzw. auskerbt.

Die Bremseinrichtung ist bevorzugtermaßen als feststehende Bremsscheibe ausgebildet, deren Außenumfang vom Innenumfang der ringförmig ausgebildeten Außentrommelscheibe unter Ermöglichung einer
reibungsbehafteten
relativen Drehung zwischen beiden umschlossen wird. Bei einer bevorzugten Ausführungsform der Erfindung ist die Bremsscheibe als elastischer Ring ausgebildet, der mindestens einen radialen Spalt aufweist, den eine bezüglich ihre Druckkraft einstellbare Druckfeder aufzuweiten versucht. Dabei ist nur der auf der einen Seite des Spaltes liegende Bereich des Bremsringes ortsfest gehalten, während der auf der anderen Seite des Spaltes befindliche Bereich des Bremsringes in federnd nachgiebiger Weise vom ortsfest gehaltenen Bereich wegdrückbar ist. Hierbei ist die Anordnung so gewählt, daß der Außentrommelring in der dem Abwickelvorgang entsprechenden Drehrichtung den nichtortsfest gehaltenen Bereich des Bremsringes unter Vergrößerung des Spaltes mitzunehmen versucht, und zwar unter Unterstützung durch die Druckfeder. Bei Abwickeldrehrichtung wird somit die Bremskraft der Bremsscheibe erhöht. Bei Aufwickeldrehrichtung dagegen versucht der Außentrommelring den nicht ortsfest gehaltenen Bereich der Bremsscheibe mitzunehmen und den Spalt zu verringern, so daß in Aufwickeldrehrichtung die Bremskraft der Bremsscheibe verringert ist.

Auch die Wirkflächen zwischen Außentrommelring und Bremsscheibe können zum Erzielen einer erhöhten Reibkraft wieder vergrößert werden, indem sie komplementär ausgebaucht bzw. ausgekerbt werden.

Bevorzugtermaßen sind die beiden an der Rutschkupplung und/oder der Bremseinrichtung beteiligten Wirkflächen aus unterschiedlich hartem Material hergestellt. Dies führt zu dem Vorteil, daß ein Abrieb des weicheren Materials entsteht und dieser Abrieb wie ein Schmiermittel zwischen den aufeinander reibenden Flächen wirkt. Ein besonders bevorzugtes Material für die Bremsscheibe, den Außentrommelring und den Treibradring ist Niederdruckpolyethylen (HD-PE für High Density Polyethylen), wobei
reibend zusammenwirkende Bauteile aus unterschiedlich hartem HD-PE hergestellt sind. Vorzugsweise wird ein Shore-Härteverhältnis von 95:80 verwendet.
Es werden also entweder der Treibradring und die Bremsscheibe aus weicherem und der Außentrommelring aus härterem HD-PE gemacht oder umgekehrt.

Bei einer besonders bevorzugten Ausführungsform der Erfindung ist die Innentrommel bezüglich der Außentrommel exzentrisch angeordnet. Dies führt zu dem Vorteil, daß bei einer Außentrommel mit bestimmtem Durchmesser ein größerer Leitungsanordnungsabschnitt zwischen Innentrommel und Außentrommel auf- und abgewickelt oder bei vorgegebener Länge dieses Leitungsordnungsabschnittes der Außentrommeldurchmesser kleiner gemacht werden kann als bei zentrischer Anordnung der Innentrommel bezüglich der Außentrommelachse.

Bevorzugtermaßen ist die mindestens eine Bremsscheibe auf dem Außenumfang der Innentrommel angeordnet.

Es ist von Vorteil, die Mantelfläche der Innentrommel und/oder der Außentrommel mindestens im Bereich der Leitungsdurchführöffnung im Querschnitt schneckenförmig auszubilden, wobei die benachbarten Schneckenlagen einen Abstand voneinander haben, der in etwa der Dicke der Leitungsanordnung entspricht. Bei solcher Ausbildung kann die Leitungsanordnung aus der Innentrommel bzw. aus der Außentrommel mit einer Krümmung entsprechend der Krümmung der Innentrommel bzw. der Außentrommel herausgeführt werden. Da bei dieser Ausgestaltung der Trommeln die Leitungsanordnung tangential aus der Innentrommel bzw. der Außentrommel herausgeführt wird und nicht radial, ist vermieden, daß die Leitungsanordnung an den Durchführstellen der Trommeln einen die Leitungsanordnung belastenden kleinen Krümmungsradius und besonders bei mehreren übereinanderliegenden Wickellagen der Leitungsanordnung hügelartige Verwerfungen aufweist.

Handelt es sich um eine elektrische Leitungsanordnung, ist diese in besonders bevorzugter Weise als Flachkabel ausgebildet. Sind mehrere Leiter in dem Kabel vorhanden, kann ein Teil dieser Leiter aus der einen offenen Stirnseite und der Rest der Leiter aus der anderen offenen Stirnseite der Innentrommel herausgeführt werden, um diese Leiter mit der feststehenden Einrichtung zu verbinden. Bevorzugtermaßen ist der diese Leiter aufnehmende Innenraum der Innentrommel mit einem Fixierungsmittel vergossen. Vorzugsweise ist die Leitungsanordnung auch im Bereich der Leiterdurchführöffnung der Außentrommel mit dieser verklebt.

Bei einer besonders bevorzugten Ausführungsform der Erfindung dient ein lineares oder gekrümmtes Tragelement für die hin- und herbewegliche Einrichtung gleichzeitig als Ablagebahn für die abgewickelte Leitungsanordnung und als Abrollschiene für die Treibradeinrichtung, wobei die Schiene, bzw. bei der Verwendung eines Treibrades auf jeder Stirnseite der Außentrommel, die Schienen, neben bzw. beidseits der Leitungsablagebahn soweit hochstehen, daß bei auf der Schiene bzw. den Schienen ablaufender Treibradeinrichtung der Außenmantel der Außentrommel auch bei maximaler Anzahl von Wicklungslagen der Leitungsanordnung frei über der Leitungsablagebahn und zwischen den Schienen drehen kann.

Zur rutschfreien Abrollbewegung des Treibradringes auf der Abrollbahn kann man den Treibradring formschlüssig mit der Abrollbahn koppeln, wobei der Treibradring z.B. als Zahnrad und die Abrollbahn als lineare bzw. gekrümmte Zahnstange ausgebildet ist. Die Abrollkopplung kann auch mittels Kette, Zahnriemen oder dergleichen erfolgen.

Um einerseits beim Aufwickeln Antriebskraft von dem Treibradring auf die Außentrommel übertragen zu können und andererseits beim Abwickeln, bei welchem der Antrieb über das Abziehen des Kabels geschieht, keinen zuzätzlichen zu zu hoher Drehgeschwindigkeit führenden Antrieb über den Treibradring zu erhalten, ist
eine besonders bevorzugte Ausführungsform der Erfindung mit einer nur in Abwickeldrehrichtung freigebenden Freilaufeinrichtung ausgestattet, die gemäß Anspruch 34 ausgebildet sein kann.

Während der Auf- und Abwickelvorgänge können in der Leitungsanordnung Zug- und Schubkräfte wirksam werden, die einerseits durch Krümmungsradiusänderungen während des Auf- und Abwickelvorgangs verursacht werden und andererseits dadurch, daß der Außenkrümmungsradius und der Innenkrümmungsradius der Leitungsanordnung aufgrund von deren Dickenabmessung unterschiedlich sind. Diese Unterschiede zwischen Außenkrümmungsradius und Innenkrümmungsradius wirken sich insbesondere in dem zwischen Außentrommel und Innentrommel befindlichen Innenbereich der Leitungsanordnung aus, wo die Leitungsanordnung in Spiralform mit engem Krümmungsradius gewickelt wird. Solche Unterschiede zwischen Außenkrümmungsradius und Innenkrümmungsradius wirken sich auch bereits bei Flachkabeln erheblich aus. Verschärft werden kann die Situation dadurch, daß die einzelnen Leiter in dem Außenmantel des Flachkabels nicht alle exakt in der neutralen Zone zwischen den Zugkräften auf der Außenseite und den Stauchungskräften auf der Innenseite des Flachkabels liegen sondern durch Produktionstoleranzen außerhalb dieser neutralen Zone liegen können. Im Außenbereich der Leitungsanordnung sind zwar die Krümmungsradien nicht so klein wie im Innenbereich der Leitungsanordnung. Dort treten aber bei Verwendung der Wickelvorrichtung für einen C-Bogen nicht nur starke Krümmungsradiusänderungen auf sondern auch eine Krümmungsradiusumkehr, wenn die Leitungsanordnung von der relativ engen Wickelkrümmung auf der Außentrommel auf die entgegengesetzt gerichtete Krümmung auf dem C-Bogen umlenken muß. Die geschilderten Einflüsse können zu einer relativ schnellen Beschädigung oder gar Zerstörung der Leitungsanordnung und damit zu Störungen bzw. Ausfällen der an die Leitungsanordnung angeschlossenen Einrichtungen führen.

Bei einer besonders bevorzugten Ausführungsform der Erfindung werden diese schädlichen Einflüsse dadurch ausgeschaltet, daß gemäß Anspruch 35 nur der Außenbereich der Leitungsanordnung mit einem Außenmantel versehen ist, daß die Leitungen im Innenbereich der Leitungsanordnung mittels einer Bündelungseinrichtung lose in etwa der Bündelform gehalten werden, die sie im Außenbereich aufgrund der Einbettung in den Außenmantel haben, wobei die einzelnen Leitungen gegeneinander und gegenüber der Bündelungseinrichtung verschiebbar sind, und daß die Leitungsanordnung derart durch die Leitungsdurchführöffnungen geführt ist, daß die einzelnen Leitungen im Bereich der Außentrommeldurchführöffnung bezüglich des Außenmantels und im Bereich der Innentrommeldurchführöffnung bezüglich dieser in ihrer Längsrichtung verschiebbar sind.

Ist die Leitungsanordnung durch ein Flachkabel mit mehreren nebeneinander angeordneten elektrischen Leitern gebildet, umfaßt die Bündelungseinrichtung bevorzugtermaßen ein Halteband mit etwa der Breite des Flachkabels, wobei das Halteband aus einem im wesentlichen nichtelastisch biegbaren Material besteht, im Bereich sowohl der Außentrommeldurchführöffnung als auch im Bereich der Innentrommeldurchführöffnung festgelegt ist und mit einer Vielzahl von über seine Länge und Breite verteilten Löchern versehen ist, durch welche hindurch die einzelnen Leiter in das Halteband in Längsrichtung eingeflochten sind.

Man kann das Halteband auch mit einer der Anzahl der Leiter entsprechenden Zahl von kanalartigen Längstaschen versehen, in welchen die Leiter aufgenommen sind.

Als Folge dieser Maßnahmen kann sich jede Leitung des Innenbereichs in die für sie günstigste Krümmungszone begeben, ohne Behinderung durch einen Außenmantel oder durch andere Leitungen. Unterschiedliche Längen der einzelnen Leitungen führen nicht mehr zu schädlichen Belastungen. Es ist ein Längenausgleich der einzelnen Leitungen dadurch ermöglicht, daß sie sich um den erforderlichen Längenbetrag gegenüber anderen Leitungen der Leitungsanordnung verschieben, was ihnen die lose Durchführung durch die Innentrommeldurchführöffnung erlaubt. Da im Außenbereich der Leitungsanordnung die einzelnen Leitungen und der Außenmantel nicht mehr beiden Endes aneinander festgeklemmt sind, kann sich der Außenmantel relativ zu den Leitungen des Außenbereichs frei verschieben und können sich Spannungskräfte aufgrund des Unterschiedes zwischen dem Außenkrümmungsradius und dem Innenkrümmungsradius des Außenmantels leichter im Außenmantel selbst abbauen, ohne die Leitungen übermäßig zu belasten.

Bei Verwendung der Wickelvorrichtung im Zusammenhang mit einem C-Bogen kann man Wickelkrümmungsänderungen des Außenbereichs der Leitungsanordnung beim Abwickeln von der Außentrommel auf den C-Bogen bzw. beim Aufwickeln des Außenbereichs von dem C-Bogen auf die Außentrommel dadurch vermeiden, daß man die Außentrommel senkrecht zu ihrer Drehachse um 180° gedreht über dem C-Bogen anordnet. Dann wird die Leitungsanordnung vom oberen Scheitelpunkt der Außentrommel auf den C-Bogen abgewickelt, wobei der auf der Außentrommel und der auf dem C-Bogen befindliche Teil der Leitungsanordnung in dieselbe Richtung gekrümmt sind.

Die Erfindung sowie weitere Aufgaben- und Merkmalsaspekte der Erfindung werden nun anhand einer bevorzugten Ausführungsform näher erläutert. In den Zeichnungen zeigen:
- Figur 1: eine perspektivische Schrägdarstellung, teilweise aufgeschnitten, einer erfindungsgemäßen Wickelvorrichtung;
- Figur 2: eine Schnittansicht der in Fig. 1 gezeigten Wickelvorrichtung ohne Leitungsanordnung bei axialem Schnitt;
- Figur 3: eine Schnittansicht einer Außentrommel der in Fig. 1 gezeigten Wickelvorrichtung;
- Figur 4: eine Schnittansicht einer Innentrommel der in Fig. 1 gezeigten Wickelvorrichtung;
- Figur 5: eine Bremsvorrichtung, teilweise aufgeschnitten, der in Fig. 1 gezeigten Wickelvorrichtung;
- Figur 6: eine modifizierte Ausführungsform der in Figur 1 gezeigten Wickelvorrichtung;
- Figur 7: ein konfektioniertes Flachkabel bevorzugter Art zusammen mit einer vereinfacht dargestellten Innentrommel der Wickelvorrichtung;
- Figur 8: eine vereinfacht dargestellte Wickelvorrichtung gemäß der Erfindung mit einem Flachkabel gemäß Figur 7 und;
- Figur 9: eine schematische, vereinfachte Darstellung des Übergangs von Außentrommel und außerhalb dieser befindlichem Außenbereich der Leitungsanordnung.

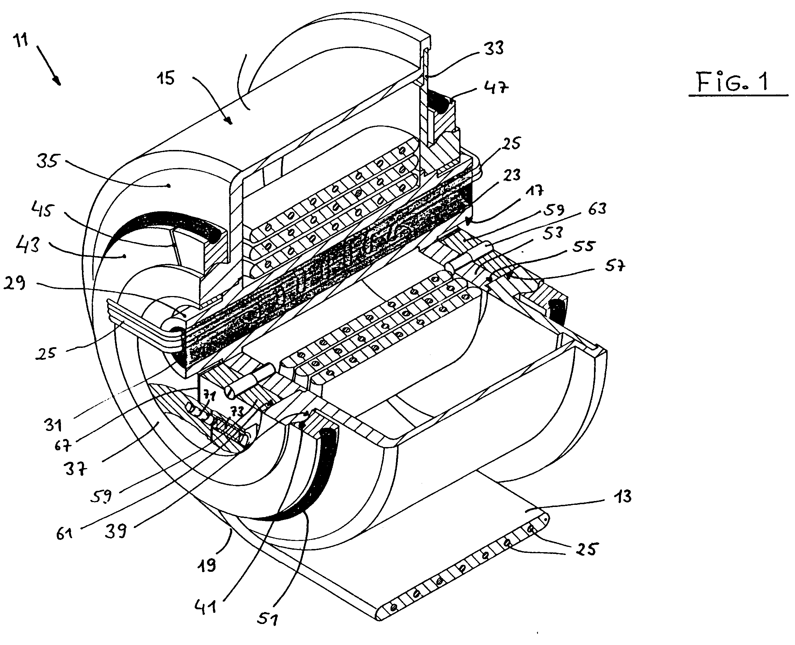

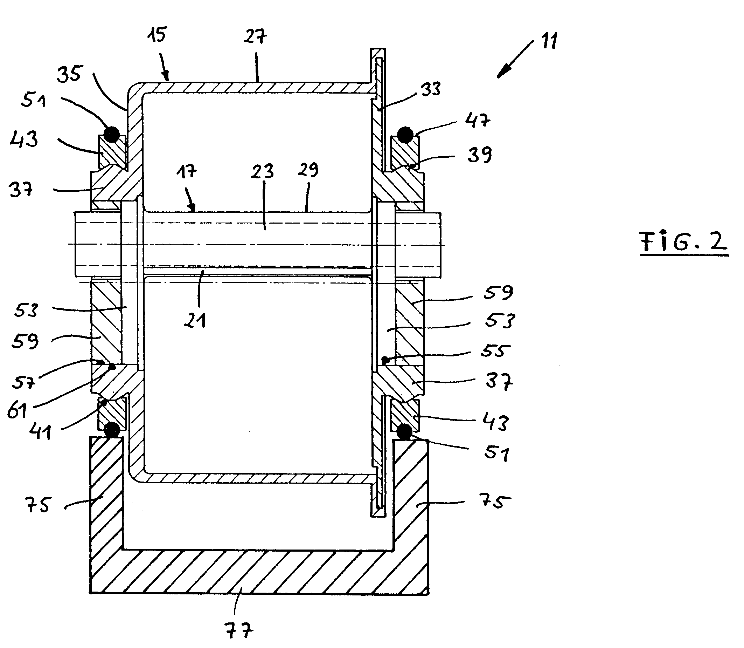

Figur 1 zeigt eine Wickelvorrichtung 11, die zum Auf- und Abwickeln eines Flachkabels 13 vorgesehen ist. Zu diesem Zweck weist die Wickelvorrichtung 15 eine um ihre Längsachse drehbare Außentrommel 15 und eine in deren Innenraum bezüglich der Außentrommelachse exzentrisch angeordnete, ortsfeste Innentrommel 17 auf. Das Flachkabel 13 ist durch eine äußere Kabeldurchführöffnung 19 von außerhalb der Außentrommel 15 in den Zwischenraum zwischen der Außentrommel 15 und der Innentrommel 17 geführt, wo es in mehreren Lagen um die Innentrommel 17 herumgewickelt ist. Die innere Lage ist durch eine innere Kabeldurchführöffnung 21 (Fig. 2) durch die Wand der inneren Trommel 17 hindurch in deren Innenhohlraum 23 geführt. Das Flachkabel 13 enthält mehrere elektrische Leiter 25, von denen ein Teil durch die in Fig. 1 linke offene Stirnfläche und der Rest durch die in Fig. 1 rechte offene Stirnfläche der Innentrommel 17 nach außen geführt ist. Die äußere Kabeldurchführöffnung 19 und möglicherweise auch die innere Kabeldurchführöffnung 21 sind je so gestaltet, daß das Flachkabel tangential aus dem Außenmantel 27 der Außentrommel 15 bzw. dem Innenmantel 29 der Innentrommel 17 austreten kann. Der die seitlich herausgeführten elektrischen Leiter 25 enthaltende Innenhohlraum 23 ist mit einer Vergußmaße 31 ausgefüllt, um die feststehenden Enden der elektrischen Leiter 25 zu fixieren.

Die Außentrommel 15 ist bis auf einen Deckel 33 auf der in Fig. 1 rechten Seite einstückig ausgebildet. Sowohl am Deckel 33 als auch an der in Fig. 1 linken Stirnfläche ist je ein Außentrommelring 37 angeformt. Die Außenumfangsfläche 39 eines jeden Außentrommelringes 37 ist mit einem ausgebauchten Profil versehen. Die Außenumfangsflächen 39 sind je von der Innenumfangs fläche 41 eines Treibradringes 43 umschlossen. Die Innenumfangsfläche 41 ist mit einem eingekerbten Profil versehen, das zu dem ausgebauchten Profil der Außenumfangsfläche 39 komplementär ist. Jeder Treibradring 43 weist einen schrägverlaufenden Radialspalt 45 auf. Die Treibradringe 43 bestehen aus einem elastischen Material. Die Außenumfangsumfläche 47 des Treibradringes weist eine Rundnut 49 auf, in der sich ein Laufring 51 aus elastischem Material befindet.

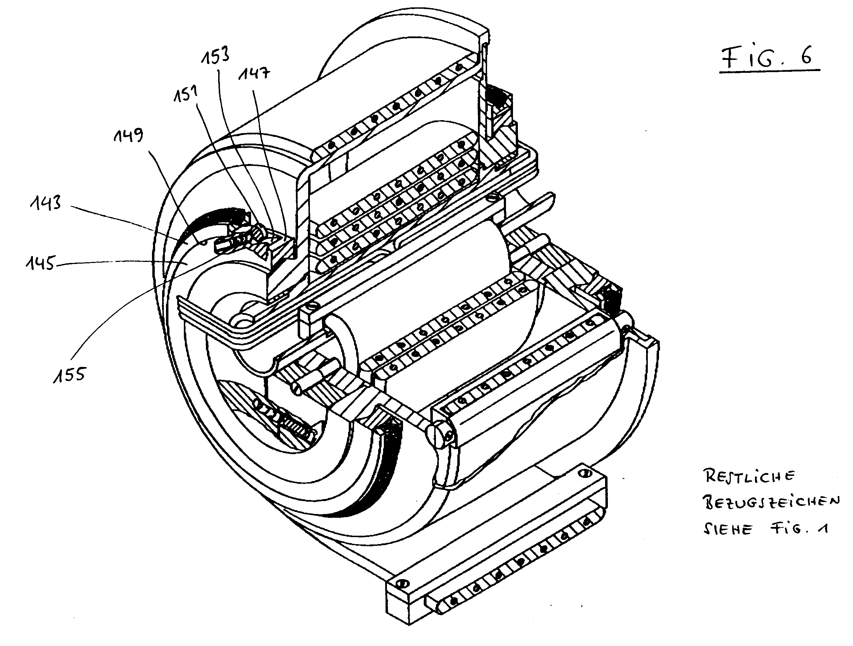

An der Innentrommel 17 sind in der Nähe ihrer axialen Enden radiale Scheiben 53 einstückig angeformt, deren Außenumfangsflächen je von einem axialen Teil der Innenumfangsfläche 57 des Außentrommelringes 37 umschlossen ist. In dem verbleibenden axialen Bereich der Innenumfangsfläche 57 eines jeden Außentrommelringes 37 ist eine Bremsscheibe 59 angeordnet, deren Außenumfangsfläche 61 mit dem zugehörigen axialen Teil der Innenumfangsfläche 57 des Außentrommelrings 37 in Reibeingriff steht. Jede Bremsscheibe 59 ist mittels einer Schraube 63 an der benachbarten radialen Scheibe 53 der Innentrommel 17 festgeschraubt. Die Bremsscheiben 59 werden dadurch ortsfest gehalten. In Fig. 1 sind die Schrauben 63 in den aufgeschnittenen Bereichen der radialen Scheiben 53 und der Bremsscheibe 59 gezeichnet, um die Schrauben 63 überhaupt darstellen zu können. In Wirklichkeit gehören sie allerdings auf die andere Seite der Außentrommelachse, um die erwünschte Funktion der Bremseinrichtung zu erzielen (siehe Fig. 5). Wie am besten in Fig. 5 zu sehen ist, weisen die Bremsscheiben 59 je ein exzentrisches Loch 65 auf, durch welches das zugehörige axiale Ende der Innentrommel 17 hindurchgeführt ist. Die Bremsscheiben 59 sind somit als exzentrische Ringe ausgebildet. Sie bestehen aus elastischem Material und weisen an der radial dicksten Stelle des exzentrischen Ringes einen radialen Bremsscheibenspalt 67 auf. In radialer Nähe zum Außenende des Bremsscheibenspaltes 65 ist in die Bremsscheibe eine durchmesserparallele Sacklochbohrung 69 eingebracht. In diese ist eine in Fig. 1 angedeutete Schraubenfeder 71 eingesetzt, die mit einer Einstellschraube 73 in der Sacklochbohrung 69 gehalten wird und deren Druckkraft durch mehr oder weniger tiefes Einschrauben der Einstellschraube 73 eingestellt werden kann. Die Schraubenfeder 71 versucht die beidseits des Bremsscheibenspaltes 67 befindlichen Bereiche der Bremsscheibe 59 vom Bremsscheibenspalt 67 wegzudrücken, d.h., den Bremsscheibenspalt 67 zu verbreitern.

Die beiden Laufringe 51 laufen auf je einer Schiene 75 (Fig. 2), die von einem Tragelement 77 hochstehen. Der Raum zwischen den Schienen 75 und der Bodenfläche des Tragelementes 77 ist so bemessen, daß sich die Außentrommel 15 mitsamt den maximal darauf gewickelten Flachkabellagen frei drehen kann.

Die Innenumfangsfläche 41 eines jeden Treibradringes 43 und die Außenumfangsfläche 39 des je zugehörigen Außentrommelringes 37 stehen in Reibeingriff zueinander und bilden eine Rutschkupplung zwischen dem Treibradring 43 und dem Außentrommelring 37. Die Rutschkupplungskraft zwischen beiden hängt einerseits von den
Materialien
von Treibradring 43 und Außentrommelring 37 und andererseits von der Kraft, mit welcher der Laufring 51 den Treibradring 43 umschließt, ab.

Die Außenumfangsfläche 61 einer jeden Bremsscheibe 59 und der die Bremsscheibe 59 umschließende axiale Teil der Innenumfangsfläche 57 des je zugehörigen Außentrommelringes 37 stehen in Bremsreibeingriff miteinander. Die von der Bremsscheibe 59 auf den zugehörigen Außentrommelring 37 ausgeübte Bremskraft hängt einerseits von der Druckkraft der Schraubenfeder 71 ab und andererseits davon, ob der nicht festgeschraubte Teil der Bremsscheibe 59 bei der jeweils vorhandenen Drehrichtung der Außentrommel 15 und damit des Außentrommelringes 37 aufgrund von Reibungsmitnahme in den Bremsscheibenspalt 67 vermindernder Weise zum festgeschraubten Bereich der Bremsscheibe 59 gedrückt oder in den Bremsscheibenspalt 67 verbreiternder Weise von dem festgeschraubten Bereich der Bremsscheibe 59 weggezogen wird.

Die Materialien der beiden an der Rutschkupplung bzw. der Bremseinrichtung beteiligten Komponenten der Wickelvorrichtung 11 weisen je unterschiedliche Härte auf. Damit wird erreicht, daß aufgrund des Reibeingriffs zwischen den beiden Komponenten auf der Oberfläche der Komponente mit dem weicheren Material ein Abrieb entsteht, der auf die Reibflächen eine Schmierwirkung ähnlich wie Gleitöl ausübt. Ein bevorzugtes Material für die Trommeln 15 und 17, den Treibradring 43 und den Außentrommelring 37 ist Niederdruckpolyethylen (HD-PE), wobei beispielsweise die Außentrommel 15 und damit die Außentrommelringe 37 aus härteren HD-PE und die Bremsscheibe 59 und der Treibradring 37 aus weicherem HD-PE bestehen. Die Auswahl von härterem und weicherem HD-PE kann aber auch umgekehrt sein.

Die Laufringe 51 bestehen aus Material mit hohem Reibungskoeffizienten, beispielsweise Gummi oder Kautschuk, derart, daß sie einerseits auf den Schienen 75 ohne Schlupf abrollen und andererseits auf dem Außenumfang des je zugehörigen Treibradringes 43 nicht verrutschen. Die Laufringe 51 sollen aus möglichst abriebfestem Material bestehen. Des weiteren sind die Laufringe 51 aus solchem elastischen Material gewählt, daß sie eine geeignete Umfangsvorspannung auf den elastischen, aufgrund des Radialspaltes 45 federnd zusammendrückbaren Treibradring 43 ausüben.

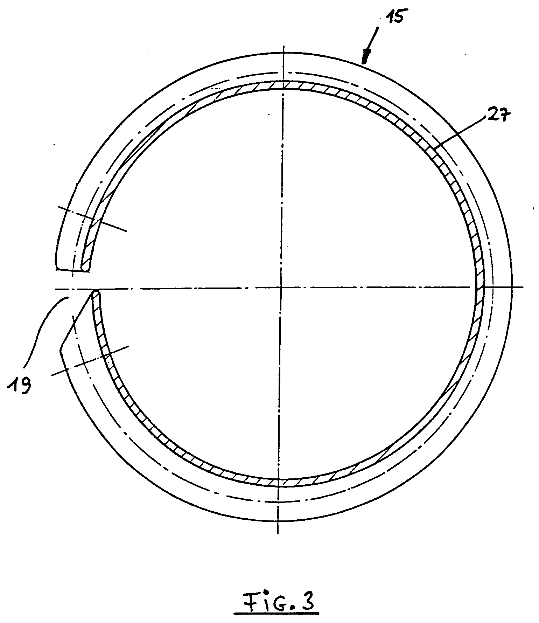

Die Querschnittsansicht in Fig. 2 zeigt die Zuordnung der zuvor beschriebenen Komponenten der Wickelvorrichtung 11 zueinander und zu dem Tragelement 77 mit den Schienen 75. In Fig. 3 ist besonders gut zu sehen, daß der Mantel der Außentrommel 15 im Bereich der äußeren Kabeldurchführöffnung 19 einen schneckenartigen Verlauf hat. Die an die äußere Kabeldurchführöffnung 19 angrenzenden Schneckenenden haben einen radialen Abstand voneinander, der in etwa der Dicke des Flachkabels 13 entspricht. Somit kann das Flachkabel 13 aus dem Innenraum zwischen den beiden Trommel 15 und 17 tangential zur Außenmantelfläche 27 herausgeführt werden.

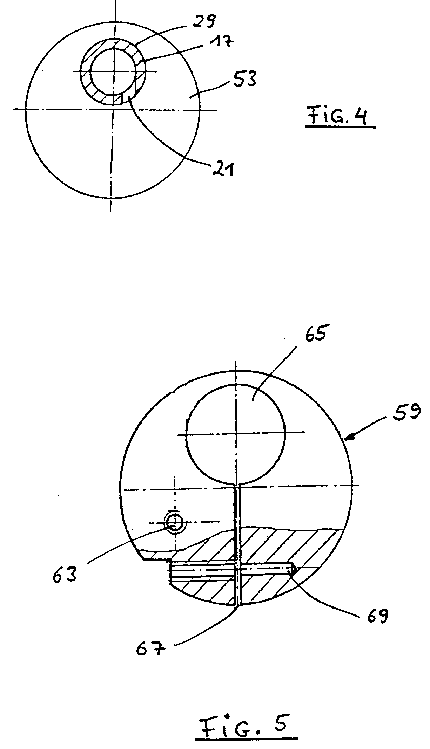

Figur 4 zeigt eine Seitenansicht der Innentrommel 17 und einer der beiden radialen Scheiben 53. Bei der in Fig. 4 gezeigten Ausführungsform ist der Innenmantel 29 der Innentrommel 17 im Bereich der inneren Kabeldurchführöffnung 21 nicht schneckenförmig ausgebildet. Eine derartige schneckenförmige Ausbildung im Bereich der inneren Kabeldurchführöffnung 21 wäre dann zu wählen, wenn das Flachkabel 13 auch aus dem Innenhohlraum 23 der Innentrommel 17 tangential herausgeführt werden sollte.

Figur 5 zeigt eine Bremsscheibe 59, teilweise im Schnitt, um die Sacklochbohrung 69 für die Schraubenfeder 71 und die Einstellschraube 73 besser zeigen zu können.

Die Arbeitsweise der beschriebenen Wickelvorrichtung 11 ist folgendermaßen.

Wenn die an dem Tragelement 77 befestigte hin- und herbewegbare Einrichtung, beispielsweise ein Röntgenstrahlungskopf eines Röntgengerätes, bewegt wird, rollen die Laufringe 51 auf den Schienen 75 ab, da die Wickelvorrichtung 11 insgesamt ortsfest gehalten ist. Die Laufringe 51 übertragen ihre Drehbewegung schlupffrei auf die Treibradringe 43. Diese versuchen über die Rutschkupplung, die zwischen den Treibradringen 43 und den Außentrommelringen 37 gebildet ist, die Außentrommelringe 37 und damit die Außentrommel 15 auf gleiche Umdrehungszahl zu bringen. Die Drehbewegung der Außentrommel 15 wird durch die Bremswirkung der Bremsscheiben 59 gebremst.

Da der Außenbereich des Flachkabels 13 an dem Tragelement 77 oder an der an dem Tragelement 77 befestigten hin- und herbewegbaren Einrichtung festgelegt ist, entspricht die Flachkabelstrecke, die bei einem Auf- oder Abwickelvorgang pro Zeiteinheit auf- bzw. abgewickelt wird, der Strecke, um welche das Tragelement 77 in dieser Zeiteinheit bewegt worden ist. Die Abrollstrecke auf jedem Laufring 51 entspricht daher der Länge, um welche das Flachkabel 13 pro Zeiteinheit auf- und abgewickelt worden ist.

Bei einem Abrollvorgang versuchen die Treibradringe 43 die Außentrommel 15 über die Rutschkupplung zwischen den Treibradringen 43 und den Außentrommelringen 37 auf gleiche Drehzahl mitzunehmen. Da der Außendurchmesser der Außentrommel 15 größer ist als der Außendurchmesser der Laufringe 51, würde dies zu einer Umfangsgeschwindigkeit der Außentrommel 15 führen, die entsprechend dem Durchmesserverhältnis von Außentrommel 15 und Laufring 51 größer ist als die Umfangsgeschwindigkeit der Laufringe 51. Da aber das Flachkabel 13 nur mit einer Umfangsgeschwindigkeit aufgewickelt werden kann, die der Umfangsgeschwindigkeit oder Abrollgeschwindigkeit der Laufringe 51 entspricht, bremst das aufzuwickelnde Flachkabel 13 die Außentrommel 15 auf eine Drehzahl, die niedriger ist als die der Laufringe 51 und damit der Treibradringe 43. Die Folge ist ein Schlupf zwischen der Außentrommel 15 und den Treibradringen 43, der durch die Rutschkupplung zwischen den Treibradringen 43 und den Außentrommelringen 37 ermöglicht wird.

Wird das Tragelement 77 in der Richtung bewegt, in welcher das Flachkabel 13 von der Außentrommel 15 abgewickelt wird, könnte sich die Außentrommel 15 ohne die Bremsscheibe 59 mit der gleichen Drehzahl wie die Treibradringe 43 drehen, so daß die Außentrommel 15 für den Aufwickelvorgang zu schnell umlaufen und damit ein straffes Aufliegen des Flachkabels 13 auf der Außentrommel 15 verhindert würde. Die Bremsscheiben 59 bremsen jedoch die Außentrommel 15 soweit ab, daß die Außentrommel 15 mit ihrer Drehzahl hinter der Drehzahl der Treibradringe 43 zurückbleiben würde, was durch die Rutschkupplung ermöglicht wird, und daß die Außentrommel 15 durch den über das Flachkabel 13 ausgeübten Zug in die richtige Abwickeldrehgeschwindigkeit gebracht wird. Mit Hilfe der Einstellschraube 73 kann die Federkraft der Schraubenfeder 71 so eingestellt werden, daß das Flachkabel 13 nur eine sehr geringe Mitnahmekraft auf die Außentrommel 15 ausüben muß.

Dreht sich die Außentrommel 15 in Abwickelrichtung, läuft die Innenumfangsfläche 57 gegen den nicht festgeschraubten Bereich der Bremsscheibe 59 und nimmt diesen aufgrund des Reibeingriffs zwischen dem Außentrommelring 37 und der Bremsscheibe 59 mit, so daß die Bremskraft erhöht wird. Läuft die Außentrommel 15 dagegen in Aufwickelrichtung um, wird der nicht festgeschraubte Bereich einer jeden Bremsscheibe 59 gegen die Kraft der Schraubenfeder 71 gegen den festgeschraubten Bereich dieser Bremsscheibe 59 gedrückt, so daß sich die Bremskraft vermindert.

Dadurch, daß bei der erfindungsgemäßen Wickelvorrichtung zwischen Treibradring 43 und Außentrommel 15 eine Rutschkupplung vorgesehen ist, wird auch auf ganz einfache Weise das Problem gelöst, daß sich während des Auf- oder Abwickelvorgangs das Verhältnis zwischen den Drehgeschwindigkeiten von Außentrommel 15 und Laufradring 43 ändert, da sich der für den Wickelvorgang maßgebliche Außendurchmesser von Außentrommel 15 und darauf befindlichem Flachkabelwickel ändert. Dies kann die Rutschkupplung problemlos ausgleichen. Während des Auf- oder Abwickelvorgangs ändert sich eben nur der Rutschschlupf zwischen Treibradring 43 und Außentrommelring 37.

Die erfindungsgemäße Wickelvorrichtung 11 stellt einen sich selbst steuernden Mechanismus dar. Alles, was für eine zufriedenstellende Funktion erfüllt sein muß, ist, daß der Außendurchmesser der Außentrommel 15 größer ist als der Außendurchmesser des Laufrings 51, damit die Außentrommel 15 immer die Chance hat, sich während des Aufwickelvorgangs schneller zu drehen als es das Zuführen des aufzuwickelnden Flachkabels 13 zuläßt. Die überschüssige Drehgeschwindigkeit der Außentrommel 15 wird dabei über die Rutschkupplung abgebaut. Auf diese Weise ist sichergestellt, daß das Flachkabel 13 immer stramm auf die Außentrommel 15 aufgewickelt wird. Für den Abwickelvorgang braucht nur die Bedingung erfüllt zu sein, daß die Außentrommel 15 von den Bremsscheiben 39 soweit abgebremst wird, daß sie versucht, hinter der für das Abwickeln erforderlichen Drehgeschwindigkeit zurückzubleiben. Damit wird ein zu schnelles Drehen der Außentrommel 15 vermieden und das Flachkabel 13 wird auch beim Abwickelvorgang straff auf der Außentrommel 15 gehalten.

Für die prinzipielle Funktionsfähigkeit der erfindungsgemäßen Wickelvorrichtung 11 reicht es aus, wenn der Außendurchmesser der Außentrommel 15 größer ist als der Außendurchmesser der Laufringe 51. Man kann allerdings eine gewisse Optimierung vornehmen. Die Kraft, welche von der Rutschkupplung übertragen werden muß, steht im Verhältnis zur Steifigkeit des zu wickelnden Flachkabels 13. Je steifer das Kabel ist, umso größer muß die von der Rutschkupplung übertragbare Kraft sein. Eine hohe Kraftübertragung kann einerseits durch Materialien mit entsprechend hohem Reibungskoeffizienten erreicht werden, was aber andererseits zu entsprechend hohem Verschleiß führen kann. Eine hohe Kraftübertragung kann aber auch dadurch erzielt werden, daß man die zusammenwirkenden Flächen groß macht. Aus diesem Grund ist es sinnvoll, den Treibradring 43 nicht zu klein zu machen, um möglichst große Kupplungswirkflächen zwischen dem Treibradring 43 und dem Außentrommelring 37 zu erhalten. Andererseits darf der Durchmesser des Laufringes 51 und damit des Treibradringes 43 nicht zu groß gemacht werden, um noch eine ausreichend hohe Geschwindigkeitsdifferenz zwischen der Umfangsgeschwindigkeit der Außentrommel 15 und der Umfangsgeschwindigkeit des Laufrings 51 sicherzustellen.

Durch die exzentrische Anordnung der Innentrommel 17 gegenüber der Außentrommelachse wird erreicht, daß eine vorbestimmte Anzahl von Auf- und Abwickeldrehungen der Außentrommel 15 mit einer geringeren Innenbereichslänge des Flachkabels 13 ermöglicht wird. Im Zwischenraum zwischen Außentrommel 15 und Innentrommel 17 muß eine bestimmte Flachkabellänge untergebracht sein, um der Außentrommel 15 eine solche Anzahl von Umdrehungen zu ermöglichen, wie sie zum Auf- und Abwickeln einer Flachkabellänge entsprechend dem maximalen Bewegungsweg der hin- und herbewegbaren Einrichtung erforderlich ist. Beim Abwickeln von Flachkabel von der Außentrommel 15 wird der zwischen den Trommeln befindliche Innenbereich des Flachkabels 13 um die Innentrommel 17 herumgewickelt. Bei der Aufwickeldrehung der Außentrommel 15 legt sich der Innenbereich des Flachkabels 13 an die Innenwand der Außentrommel 15. Bei der Aufwickelendstellung der Außentrommel 15 ist das an der äußeren Kabeldurchführöffnung 19 festgelegte Ende des Innenbereichs des Flachkabels 13 zu dem auf der Innentrommel befindlichen Flachkabelwickel hin gespannt. In der Abwickelendstellung der Außentrommel 15 ist das an der inneren Kabeldurchführöffnung 21 festgelegte Ende des Innenbereichs des Flachkabels 13 zwischen dem Innentrommelmantel und dem auf der Innenseite des Außentrommelmantels liegenden Flachkabelwickel gespannt. Ist die Innentrommel zentrisch zur Außentrommel angeordnet, ist der in der jeweiligen Wickelendstellung der Außentrommel 15 zwischen Außentrommel und Innentrommel gespannte Teil des Innenbereichs des Flachkabels 13 größer als bei exzentrischer Anordnung der Innentrommel 17 innerhalb der Außentrommel 15. Denn bei exzentrischer Anordnung ist der Minimalabstand zwischen Außentrommel 15 und Innentrommel 17 erheblich kleiner als der Minimalabstand zwischen Außentrommel 15 und Innentrommel 17 bei zentrischer Anordnung.

Hin- und herbewegliche Einrichtungen, denen mit der erfindungsgemäßen Wickelvorrichtung 11 eine problemlose Energieversorgung ermöglicht werden soll, werden häufig in Umgebungen eingesetzt, in denen es zu Ver schmutzungen kommt. Dies gilt für Roboter und Werkzeugmaschinen, gilt aber auch für Röntgengeräte, die unmittelbar am Operationsort zur Beobachtung während der Operation eingesetzt werden. Es besteht daher die Notwendigkeit, den Außenbereich des Flachkabels 13 ab und zu zu reinigen. Mit der erfindungsgemäßen Wickelvorrichtung 11 ist dies völlig unproblematisch. Alles, was man zu tun braucht, ist, die hin- und herbewegliche Einrichtung in eine Stellung zu bringen, in welcher der Außenbereich des Flachkabels 13 auf die Außentrommel 15 aufgewickelt ist, das Tragelement 77 in dieser Position unbeweglich zu halten, das Flachkabel 13 unter Überwindung der Rutschkupplungskraft von Hand von der Außentrommel 15 abzuziehen, so daß auch seine dem Tragelement 77 zugewandte Seite der Reinigung frei zugänglich ist, und dann die hin- und herbewegliche Einrichtung in die Stellung zurückzubewegen, in welcher der Außenbereich des Flachkabels 13 vollständig auf die Außentrommel 15 aufgewickelt ist. Da bei dieser Zurückbewegung das Flachkabel 13 nicht stramm gespannt ist, sondern in Form einer lockeren Schlaufe von der Außentrommel 15 weghängt, kann die Außentrommel 15 bei dieser Rückbewegung die Drehgeschwindigkeit annehmen, die sie bei starrer Kopplung zwischen Treibradring 43 und Außentrommelring 37 einnähme, so daß das Flachkabel 13 wieder stramm auf die Außentrommel 15 aufgewickelt wird.

Durch entsprechende Auswahl der an der Rutschkupplung beteiligten Materialien, der Breite und des Durchmessers der an der Rutschkupplung beteiligten Flächen und des Verhältnisses der Außendurchmesser von Außentrommel 11 und Laufring 51 kann eine für die jeweilige Anwendung günstigste Lösung geschaffen werden.

Figur 6 zeigt die Wickelvorrichtung nach Figur 1 in modifizierter Form, nämlich mit einer Freilaufeinrichtung, die nur in der Abwickeldrehrichtung bezüglich des Außenbereichs der Leitungsanordnung freigebend wirkt. Damit wird erreicht, daß beim Aufwickelvorgang die erwünschte Kraftübertragung von dem Treibradring auf die Außentrommel stattfinden kann, während beim Abwickelvorgang ein Antrieb durch den Treibradring zusätzlich zu dem Antrieb durch das abgezogene mit Hilfe der Freilaufrichtung verhindert wird. Dies führt dazu, daß die Bremseinrichtung nur die vom abgezogenen Kabel erzeugte Antriebskraft der Außentrommel abzubremsen braucht und nicht noch zusätzlich eine über den Treibradring erzeugte Antriebskraft.

Zu diesem Zweck ist der Treibradring 43 von Figur 1 in zwei konzentrische Teilringe 143 und 145 untergeteilt, die sich in Abwickeldrehrichtung der Wicklungsvorrichtung frei gegeneinander verdrehen können, während in Aufwickeldrehrichtung eine Verriegelung zwischen den beiden Teilringen 143 und 154 auftritt, so daß sie sich im wesentlichen als Einheit drehen, vergleichbar dem einteiligen Treibradring 43 bei der Ausführungsform nach Fig.1.

Zu diesem Zweck ist in die Umfangsfläche eines der beiden Teilringe, bei der Ausführungsform nach Figur 6 in die Umfangsfläche 147 des Teilringes 145, eine Anzahl von Schrägflächenmulden 153 eingelassen, in denen je eine kugel- oder scheibenartige Hemmrolle 151 eingesetzt ist. Die Schrägflächenmulde 153 und die Hemmrolle 151 sind so dimensioniert, daß die Hemmrolle 151 dann, wenn sie sich an der tiefsten Stelle der Schrägflächenmulde 153 befindet, nicht über die Ringumfangsflache 147 des Teilrings 145 hinausragt. Ist sie dagegen die Schrägfläche der Schrägflächenmulde 153 ein Stück hochgelaufen, ragt sie über die Ringumfangsfläche 147 hinaus, so daß sie die Bewegungsbahn der Ringumfangsfläche 149 des Teilrings 143 schneidet. Als Folge davon wird eine Relativbewegung zwischen den beiden Teilringen 143 und 145 zunächst gebremst und schließlich ganz unterbunden.

Das Hinauf- bzw. Hinabbewegen der Hemmrolle 151 auf der Schrägfläche der Schrägflächenmulde 153 geschieht dadurch, daß die Hemmrolle 151 von der Ringumfangsfläche 149 des Teilrings 143 relativ zur Schrägfläche der Schrägflächenmulde 153 verrollt wird. Damit die Hemmrolle 151 auch aus der tiefsten Stelle der Schrägflächenmulde 153, in welcher sie von der Ringumfangsfläche 149 des Teilrings 143 nicht erfaßt wird, wieder die Schrägfläche der Schrägflächenmulde 153 hinauf bewegt werden kann, steht sie unter einer sie in dieser Bewegungsrichtung vorspannenden Kraft einer Druckfeder 155.

Ist die Außentrommel auf beiden axialen Seiten mit je einem Treibradring 43. versehen, ist jedem dieser Treibradringe 43 eine Freilaufeinrichtung gemäß Figur 6 zuzuordnen.

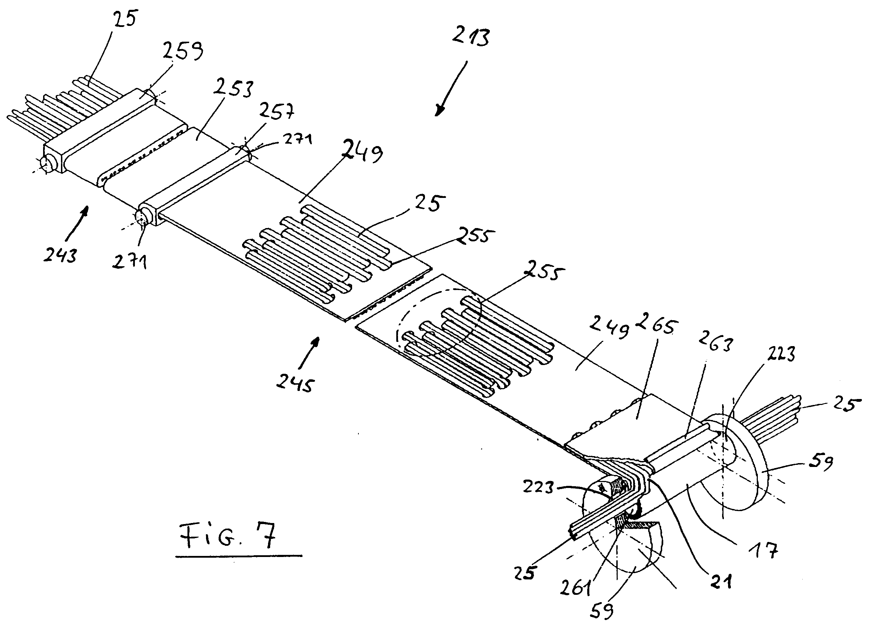

Anhand der Figuren 7 bis 9 werden nun Einzelheiten einer besonders bevorzugten Ausführungsform erläutert, bei welcher die Leitungsanordnung belastende oder gar zerstörende Kräfte aufgrund von Krümmungswechseln der Leitungsanordnung und unterschiedlichen Krümmungsradien auf der Außenseite und der Innenseite der gekrümmten Leitungsanordnung vermieden werden.

Zunächst wird anhand von Figur 7 ein in unterbrochener Zeichnungsweise dargestelltes konfektioniertes Flachkabel 213 dargestellt, das an einer vereinfacht dargestellten Innentrommel 17 montiert ist. Die Leiter 25 des Flachkabels 213 liegen nebeneinander in einer Reihe und sind je mit einer Primärisolierung versehen. Im Außenbereich 243 des Flachkabels 213 sind die Leiter 25 in einen Außenmantel 253 aus einem flexiblen Isolierstoff aus Gummi oder einem gummiähnlichen Kunststoff eingebettet. Im Innenbereich 45 sind die Leiter 25 frei von dem Außenmantel 253. Mittels eines Haltebandes 249 werden sie im wesentlichen in einer Lage gehalten, die ihrer Anordnung im Außenmantel 253 entspricht. Das Halteband 249 ist mit Löchern 255 versehen, die über die Breite und Länge des Haltebandes 249 so verteilt sind, daß die Leiter 25 in ihrer Längsrichtung mit dem Halteband 249 verflochten werden können. Bei der dargestellten Ausführungsform sind durch ein Loch 255 je zwei Leiter 25 gefädelt. Man könnte auch mehrere Leiter 25 oder nur einen Leiter 25 durch jedes Loch fädeln.

Das Halteband 249 besteht bei der bevorzugten Ausführungsform aus einem weichen Fliesmaterial, das so nachgiebig ist, daß sich bei den Auf- und Abwickelvorgängen jeder Leiter 25 seine neutrale Phase und Lage selber suchen kann. Daß unterschiedliche Leiter 25 unterschiedliche neutrale Phasen oder Lagen aufsuchen, ist bedingt durch Durchmesserschwankungen der einzelnen Leiter 25 oder durch zu lose oder zu stramm eingeklemmte Leiter 25. Auf einer
Seite ist das Fliesmaterial mit einer Kunststoffschicht oder -folie guter Gleitfähigkeit versehen. Als Material für diese Folie eignet sich beispielsweise Polytetrafluoräthylen (PTFE).

Im Übergangsbereich zwischen Außenbereich 243 und Innenbereich 245 sind die Leiter 25 und das Halteband 245 mittels einer ersten Klemmleiste 257 festgeklemmt. Der Außenmantel 253 ist nicht in die Klemmleiste 257 miteingeklemmt sondern endet vor dieser. Am anderen Ende des Außenbereichs 243 ist auf den Außenmantel 253 eine zweite Klemmleiste 259 aufgeklemmt. Aus diesem Ende des Außenmantels 253 ragen die Leiter 25 so weit vor, daß sie an eine mitbewegliche Verbinderanordnung 31 oder direkt an eine über das Flachkabel 213 anzuschließende hin- und herbewegliche Einrichtung angeschlossen werden können.

Die Leiter 25 des Innenbereichs 245 stehen so weit über das freie Ende des Haltebandes 249 über, daß sie durch die Innentrommeldurchführöffnung 21 hindurch und aus dem Inneren der Innentrommel 17 seitlich herausführbar und an eine feststehende Verbinderanordnung oder direkt an eine feststehende Einrichtung anschließbar sind.

In Figur 7 ist die Montage des freien Endes des Innenbereichs 245 dargestellt. Diese Figur zeigt die Innentrommel 17 mit ihren vereinfacht dargestellten Seitenscheiben 59, teilweise aufgeschnitten. Die freien Enden der Leiter 25 sind mittels der Innentrommeldurchführöffnung 21 von außerhalb der Innentrommel 17 in deren Innenraum 261 geführt. Ein Teil der Leiter 25 ist aus einer in Figur 7 linken seitlichen Öffnung 223 der in Figur 7 linken Seitenscheibe 59 herausgeführt. Die restlichen Leiter 25 sind über eine in Figur 7 rechte seitliche Öffnung 23 aus der in Figur 7 rechten Seitenscheibe 59 herausgeführt.

Was in Figur 7 nicht gezeigt ist, um diese Figur nicht zu überfrachten und zu undeutlich zu machen, ist, daß die Leiter 25 lose durch die Innentrommeldurchführöffnung 21 geführt und in ein Harz eingegossen sind, das nicht an den Leitern 25 haftet sondern diese nur in Position hält. Dadurch sind die Leiter 25 relativ zu dem Harz und damit relativ zur Innentrommeldurchführöffnung 21 in ihrer Längsrichtung verschiebbar, so daß die Leiter 25 gegebenenfalls nachgeben können, wenn sie zu stramm oder zu locker liegen.

Um diese Längsbeweglichkeit der Leiter 25 nicht zu behindern, ist das Halteband 249 außerhalb der Innenntrommeldurchführöffnung 21 an dem Innenzylinder 17 festgelegt, und zwar mittels einer Halteleiste 263. Bei der in Figur 7 gezeigten Ausführungsform ist mit der Halteleiste 263 ein Fliesstück 265 festgehalten, das schützend über den freiliegenden Leitern 25 liegt.

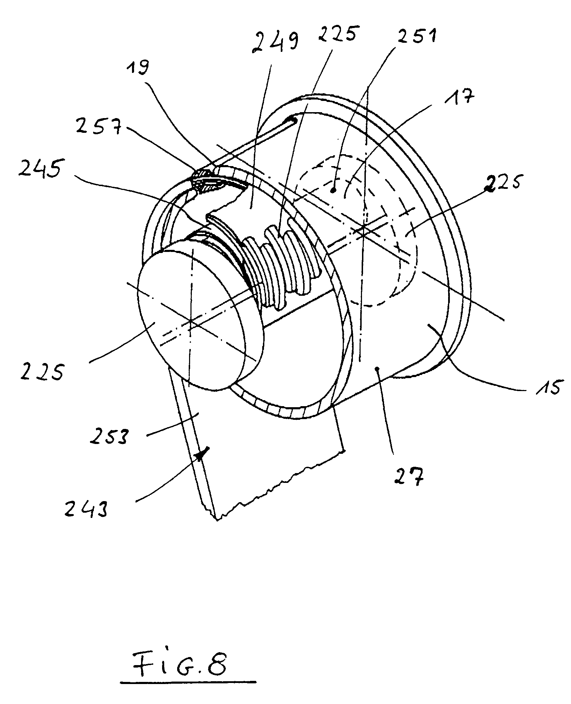

In Figur 8 sind die Außentrommel und die Innentrommel zusammen mit einem Teil des Außenbereichs 243 und einem Teil des Innenbereichs. 245 des Flachkabels 213 schematisch und nur teilweise dargestellt. Beispielsweise sind die Antriebs-, Brems- und Freilaufelemente der Wickelvorrichtung gemäß Figuren 1 und 6 nicht gezeigt und es sind auch nicht die axialen Austrittsöffnungen der Innentrommel gezeigt.

Figur 8 zeigt einen Teil des auf dem Außenumfang 27 der Außentrommel 15 aufliegenden Außenbereichs 243 des Flachkabels 213, wo die Leiter 25 von dem Außenmantel 253 umgeben sind. An der Außentrommeldurchführöffnung 19 befindet sich die Klemmleiste 257. Im Zwischenraum zwischen Außentrommel 15 und Innentrommel 17 befindet sich in spiraliger Wicklung der Verbund aus den vom Außenmantel 253 freien Leitern 25 und dem sie in Flachbündelform haltenden Halteband 249.

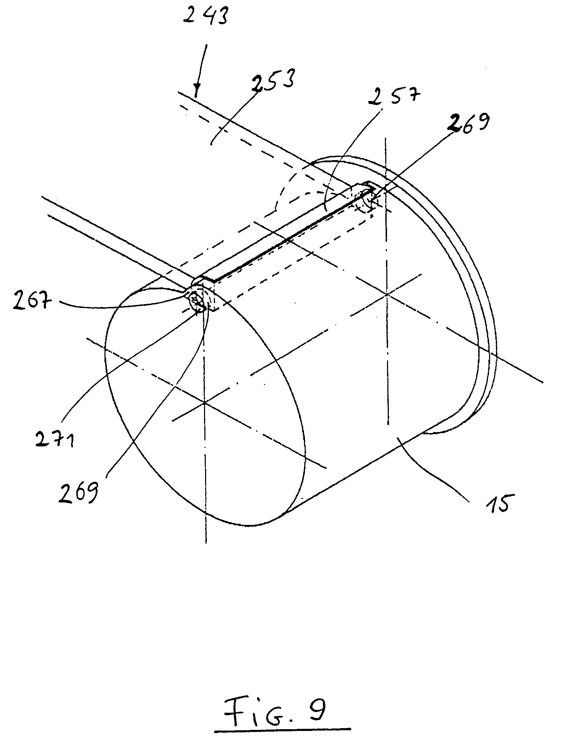

In Figur 9 ist anhand einer schematischen Darstellung der Außentrommel 15, wobei die Öffnungen für die Seitenscheiben 59 der Innentrommel 17 nicht dargestellt sind, die Befestigung der Leitungsanordnung an der Außentrommeldurchführöffnung 19 gezeigt. Aufgrund der schneckenförmigen Ausbildung des Trommelmantels der Außentrommel 15 im Bereich der Außentrommeldurchführöffnung 19 ergibt sich im Bereich der Außentrommeldurchführöffnung 19 eine radiale Stufe 267. Diese ist an beiden Stirnseiten der Außentrommel 15 je mit einem Montageloch 269 versehen. In diese Montagelöcher 269 reichen Achsstummel 271 an beiden Enden der ersten Klemmleiste 257 hinein, um diese festzuhalten.

## Patentansprüche

1. Wickelvorrichtung (11) zum Auf- und Abwickeln einer Leitungsanordnung (13), die einen Endes mit einer feststehenden Einrichtung und anderen Endes mit einer hin- und herbeweglichen Einrichtung gekoppelt ist,
mit einer ortsfesten hohlzylindrischen Innentrommel (17) und einer um diese herum drehbaren hohlzylindrischen Außentrommel (15),
wobei die Innentrommel (17) und die Außentrommel (15) je mit einer Leitungsdurchführöffnung (19, 21) zum Hindurchführen und dort Fixieren der Leitungsanordnung (13) versehen sind,
und wobei durch Drehen der Außentrommel (15) ein außerhalb der Außentrommel (15) befindlicher Außenbereich der Leitungsanordnung (13) auf deren Außenumfang (27) auf- bzw. abwickelbar und ein zwischen Außentrommel (15) und Innentrommel (17) befindlicher Innenbereich der Leitungsanordnung (13) bezüglich der Innentrommel (17) ab- bzw. aufwickelbar ist,
**dadurch gekennzeichnet**,
daß eine durch die Bewegung der hin- und herbeweglichen Einrichtung getriebene Antriebseinrichtung (51, 43) über eine Rutschkupplung (37, 43) mit der Außentrommel (15) gekoppelt und im Verhältnis zum Außentrommeldurchmesser derart dimensioniert ist, daß die Antriebseinrichtung (51, 43) die Außentrommel (15) bei rutschfreier Kupplung auf eine Umfangsgeschwindigkeit brächte, die größer wäre als die für das Wickeln des Außenbereiches der Leitungsanordnung (13) erforderliche Umfangsgeschwindigkeit der Außentrommel (15),
und daß die Außentrommel (15) unter der Bremswirkung einer Bremseinrichtung (39) steht, deren Bremskraft so dimensioniert ist, daß sie die Außentrommel (15) in Abwickeldrehrichtung auf eine Umfangsgeschwindigkeit abzubremsen versucht, die niedriger ist als die der Abwickelgeschwindigkeit des Außenbereichs der Leitungsanordnung (13) entsprechende Umfangsgeschwindigkeit der Außentrommel (15).

2. Wickelvorrichtung nach Anspruch 1,
dadurch gekennzeichnet,
daß die Antriebseinrichtung durch eine Treibradeinrichtung (51, 43) gebildet ist, die mit einer neben der Auf- und Abwickelbahn des Außenbereichs der Leitungsanordnung (13) befindlichen Abrollbahn (75) zusammenwirkt, und daß die Außentrommel (15) gegenüber der Abrollbahn (75) frei drehbar gehalten ist.

3. Wickelvorrichtung nach Anspruch 2,
dadurch gekennzeichnet,
daß an mindestens einer äußeren Stirnseite der Außentrommel (15) eine zur Außentrommel (15) koaxiale und mit dieser mitdrehende Außentrommelscheibe (37) vorgesehen ist, auf deren Außenumfang ein Treibradring (43) gelagert ist, der unter Überwindung einer Reibkraft zwischen Außentrommelscheibe (37) und Treibradring (43) relativ zur Außentrommelscheibe (37) verdrehbar ist, dessen Außendurchmesser kleiner ist als der Außendurchmesser der Außentrommel (15) und dessen Außenumfang bezüglich der Abrollbahn (75) einen einen Abrollschlupf verhindernden hohen Reibungskoeffizienten aufweist.

4. Wickelvorrichtung nach Anspruch 3,
dadurch gekennzeichnet,
daß auf dem Außenumfang des Treibradringes (43) ein Laufring (51) angeordnet ist, der sowohl bezüglich des Materials der Abrollbahn (75) als auch bezüglich des Materials des Treibradringes (43) einen einen Schlupf des Laufringes (51) verhindernden hohen Reibungskoeffizienten aufweist.

5. Wickelvorrichtung nach Anspruch 4,
dadurch gekennzeichnet,
daß sowohl der Laufring (51) als auch der Treibradring (43) aus elastischem Material bestehen, daß der Treibradring (43) mindestens einen Radialspalt (45) aufweist, und daß der Treibradring (43) von dem elastischen Laufring (51) mit einer vorbestimmten Umschließungskraft entsprechend der erforderlichen Rutschkupplungskraft auf den Außenumfang der Außentrommelscheibe (37) aufgepreßt wird.

6. Wickelvorrichtung nach mindestens einem der Ansprüche 2 bis 5,
dadurch gekennzeichnet,
daß die Außentrommelscheibe (37) ein eingekerbtes oder ausgebauchtes Außenumfangsprofil und der Treibradring (43) ein dazu komplementär ausgebauchtes bzw. eingekerbtes Innenumfangsprofil aufweist.

7. Wickelvorrichtung nach Anspruch 6,
dadurch gekennzeichnet,
daß die Außentrommelscheibe (37) eine konkave oder konvexe Außenumfangsfläche und der Treibradring eine dazu komplementär konvexe bzw. konkave Innenumfangsfläche aufweist.

8. Wickelvorrichtung nach Anspruch 6,
dadurch gekennzeichnet,
daß die Außentrommelscheibe (37) eine V-förmige Außenumfangsfläche und der Treibradring (43) eine dazu komplementär geformte V-förmige Innenumfangsfläche aufweist.

9. Wickelvorrichtung nach mindestens einem der Ansprüche 1 bis 7,
dadurch gekennzeichnet,
daß die Außentrommelscheibe als Außentrommelring (37) ausgebildet ist, dessen Innenumfang auf dem Außenumfang (61) einer dazu koaxialen, ortsfesten Bremsscheibe (59) unter Reibbremsung umläuft.

10. Wickelvorrichtung nach Anspruch 9,
dadurch gekennzeichnet,
daß die Bremsscheibe (39) mindestens einen radialen Bremsscheibenspalt (67) aufweist und daß die dem Bremsscheibenspalt (67) benachbarten Bereiche der Bremsscheibe (59) mittels einer Druckfeder (71) in den Bremsscheibenspalt (67) verbreiternder Richtung vorgespannt sind.

11. Wickelvorrichtung nach Anspruch 9,
dadurch gekennzeichnet,
daß die Druckkraft der Druckfeder (71) mittels einer Einstellschraube (73) einstellbar ist.

12. Wickelvorrichtung nach Anspruch 10 oder 11,
dadurch gekennzeichnet,
daß die Bremsscheibe (59) nur in dem in Abwickeldrehrichtung der Außentrommel (15) gesehen vor dem Bremsscheibenspalt (67) liegenden Bereich ortsfest gehalten ist, so daß der in dieser Abwickeldrehrichtung gesehen hinter dem Bremsscheibenspalt (67) befindliche Bereich der Bremsscheibe (59) infolge der Reibung zwischen Außentrommelring (37) und Bremsscheibe (59) bei in Abwickelrichtung gedrehter Außentrommel (15) unter Aufweitung des Bremsscheibenspaltes (67) und mit Unterstützung der Druckfeder (71) von dem ortsfest gehaltenen Bereich der Bremsscheibe (59) weggedrückt wird, so daß die Bremskraft der Bremsscheibe (59) bei Drehung der Außentrommel (15) in Abwickelrichtung höher ist als bei Drehung der Außentrommel (15) in Aufwickelrichtung.

13. Wickelvorrichtung nach mindestens einem der Ansprüche 9 bis 12,
dadurch gekennzeichnet,
daß die Bremsscheibe (59) als Bremsring ausgebildet ist, dessen Innenumfang auf dem Außenumfang der Innentrommel (17) angeordnet ist.

14. Wickelvorrichtung nach mindestens einem der Ansprüche 1 bis 12,
dadurch gekennzeichnet,
daß die Innentrommel (17) bezüglich der Außentrommelachse exzentrisch angeordnet ist.

15. Wickelvorrichtung nach Anspruch 14,
dadurch gekennzeichnet,
daß der kleinste radiale Abstand zwischen Innentrommel (17) und Außentrommel (15) etwa das dreifache der Leitungsanordnungsdicke beträgt.

16. Wickelvorrichtung nach Anspruch 14 oder 15,
dadurch gekennzeichnet,
daß der Bremsring (59) an der Stelle größter radialer Ringstärke mit einem radialen Bremsscheibenspalt (67) versehen ist.

17. Wickelvorrichtung nach mindestens einem der Ansprüche 1 bis 16,
dadurch gekennzeichnet,
daß die Mantelfläche der Innentrommel (17) und/oder der Außentrommel (15) mindestens im Bereich der Leitungsdurchführungsöffnungen (19, 21) im Querschnitt schneckenförmig ausgebildet ist und daß das die eine Seite der Leitungsdurchführungsöffnung (19, 21) begrenzende Schneckenende von dem die andere Seite der Leitungsdurchführungsöffnung (19, 21) begrenzenden Schneckenende einen radialen Abstand von etwa der Dicke der Leitungsanordnung (13) aufweist.

18. Wickelvorrichtung nach mindestens einem der Ansprüche 1 bis 17,
dadurch gekennzeichnet,
daß die Leitungsanordnung (13) eine elektrische Leitungsanordnung ist.

19. Wickelvorrichtung nach Anspruch 18,
dadurch gekennzeichnet,
daß die Leitungsanordnung (13) eine Mehrzahl elektrischer Leiter (25) aufweist.

20. Wickelvorrichtung nach mindestens einem der Ansprüche 1 bis 19,
dadurch gekennzeichnet,
daß die Leitungsanordnung durch ein Flachkabel (13) gebildet ist.

21. Wickelvorrichtung nach mindestens einem der Ansprüche 1 bis 20,
dadurch gekennzeichnet,
daß die Innentrommel (17) mindestens an einer Stirnseite offen ist und daß der mit der feststehenden Einrichtung koppelbare Teil der Leitungsanordnung (13) aus dem Innenhohlraum (23) der Innentrommel (17) axial durch die offene Stirnseite herausgeführt ist.

22. Wickelvorrichtung nach Anspruch 21,
dadurch gekennzeichnet,
daß die beiden Stirnseiten der Innentrommel (17) offen sind und daß von dem mit der feststehenden Einrichtung koppelnden Teil der Leitungsanordnung (13) eine erste Anzahl Leiter (25) durch die eine und die restliche Anzahl Leiter (25) durch die andere offene Stirnseite der Innentrommel (17) axial herausgeführt ist.

23. Wickelvorrichtung nach mindestens einem der Ansprüche 1 bis 22,
dadurch gekennzeichnet,
daß der Innenhohlraum (23) der Innentrommel (17) mit dem darin befindlichen feststehenden Teil der Leitungsanordnung (13) mit fixierendem Material ausgegossen ist.

24. Wickelvorrichtung nach mindestens einem der Ansprüche 9 bis 23,
dadurch gekennzeichnet,
daß der Innenumfang der Außentrommelscheibe (37) und der Außenumfang der Bremsscheibe (59) ein Umfangsprofil entsprechend einem der Ansprüche 6 bis 8 aufweisen.

25. Wickelvorrichtung nach mindestens einem der Ansprüche 3 bis 24,
dadurch gekennzeichnet,
daß auf beiden Stirnseiten der Außentrommel (15) je ein Treibradring (43) angeordnet ist,
daß längs der Auf- und Abwickelbahn des Außenbereichs der Leitungsanordnung (13) eine Schiene (75, 77) mit einem Querschnittsprofil etwa eines liegenden,
nach oben offenen C verläuft,
daß die Treibradringe (43) bzw. Laufringe (51) auf den hochstehenden Schenkeln (75) des C abrollen,
und daß die Außentrommel (15) zwischen den hochstehenden Schenkeln (75) in gegenüber der Schiene (75, 77) frei drehbarer Weise angeordnet ist.

26. Wickelvorrichtung nach mindestens einem der Ansprüche 3 bis 25,
dadurch gekennzeichnet,
daß zwischen dem Treibradring (34) und der Abrollbahn (75) eine formschlüssige Verbindung besteht.

27. Wickelvorrichtung nach mindestens einem der Ansprüche 2 bis 26,
dadurch gekennzeichnet,
daß die Abrollbahn (75) Teil eines Tragelementes (77) für die hin- und herbewegliche Einrichtung ist.

28. Wickelvorrichtung nach Anspruch 27,
dadurch gekennzeichnet,
daß das Tragelement (77) durch eine kreisbogenförmige Schiene gebildet ist, an deren mindestens einem Ende eine Einrichtung angeordnet ist, die durch kreisbogenförmiges Verschwenken der kreisbogenförmigen Schiene längs eines Kreisbogens hin- und herbewegbar ist.

29. Wickelvorrichtung nach Anspruch 28,
dadurch gekennzeichnet,
daß die Einrichtung eine Röntgenstrahlungsquelle ist.

30. Wickelvorrichtung nach mindestens einem der Ansprüche 3 bis 29,
dadurch gekennzeichnet,
daß die Außentrommelscheibe (37) und der Treibradring (43) mindestens im Bereich ihrer zusammenwirkenden Umfangsflächen aus Materialien unterschiedlicher Härte bestehen.

31. Wickelvorrichtung nach mindestens einem der Ansprüche 3 bis 30,
dadurch gekennzeichnet,
daß der Außentrommelring (37) und die Bremsscheibe (59) mindestens im Bereich ihrer zusammenwirkenden Umfangsflächen aus Materialien unterschiedlicher Härte bestehen.

32. Wickelvorrichtung nach Anspruch 30 oder 31,
dadurch gekennzeichnet,
daß der Außentrommelring (37) und der Treibradring (43) und/oder die Bremsscheibe (59) mindestens im Bereich ihrer zusammenwirkenden Umfangsflächen aus Niederdruckpolyethylen (HD-PE) unterschiedlicher Härte bestehen.

33. Wickelvorrichtung nach mindestens einem der Ansprüche 1 bis 32,
dadurch gekennzeichnet,
daß zwischen Antriebseinrichtung (51, 43) und Außentrommel (15) eine nur in der Abwickeldrehrichtung des Außenbereichs der Leitungsanordnung freigebend wirkende Freilaufeinrichtung (143, 145, 151, 153, 155) vorgesehen ist.

34. Wickelvorrichtung nach Anspruch 33,
dadurch gekennzeichnet,
daß die Freilaufeinrichtung (143, 145, 151, 153, 155) durch eine Unterteilung des Treibradringes (43) in zwei konzentrische Teilringe (143, 145) gebildet ist, zwischen deren sich gegenüberstehenden Ringumfangsflächen (147, 149) Hemmrollen (151) angeordnet sind, die auf einer in Radialrichtung geneigten Schrägflächenmulde (153) in der Ringumfangfläche (147) des einen Teilrings (145) beweglich sind und einen derartigen Durchmesser aufweisen, daß sie bei Positionierung an der tiefsten Stelle der Schrägflächenmulde (153) nicht über diese Ringumfangsfläche (147) hinausragen, jedoch im Laufe des Hochlaufens auf der Schrägfläche der Schrägflächenmulde (153) über diese Ringumfangsfläche (147) hinausragen und dadurch die freie Verdrehbarkeit der beiden Teilringe (143, 145) gegeneinander behindern, wobei die Hemmrollen (151) unter einer Federvorspannung (155) stehen, welche sie aus der tiefsten Stelle der Schrägflächenmulde (153) herauszudrücken versucht.

35. Wickelvorrichtung nach mindestens einem der Ansprüche 1 bis 34,
wobei die Leitungsanordnung eine Vielzahl von flexiblen Leitungen (25) aufweist, die gemeinsam in einem Außenmantel (253) aus flexiblem Material eingebettet sind,
dadurch gekennzeichnet,
- daß nur der Außenbereich (243) der Leitungsanordnung mit dem Außenmantel (253) versehen ist,
- daß die Leitungen (25) im Innenbereich (245) der Leitungsanordnung mittels einer Bündelungseinrichtung (249) lose in etwa der Bündelform gehalten werden, die sie im Außenbereich (243) aufgrund der Einbettung in den Außenmantel (253) haben, wobei die einzelnen Leitungen (25) gegeneinander und gegenüber der Bündelungseinrichtung (249) verschiebbar sind,
- und daß die Leitungsanordnung derart durch die Leitungsdurchführöffnungen (19, 21) geführt ist, daß die einzelnen Leitungen (25) im Bereich der Außentrommeldurchführöffnung (19) bezüglich des Außenmantels (253) und im Bereich der Innentrommeldurchführöffnung (21) bezüglich dieser in ihrer Längsrichtung verschiebbar sind.

36. Wickelvorrichtung nach Anspruch 35,
wobei ein Flachkabel (213) mit einer Reihe nebeneinander angeordneter elektrischer Leiter (25) vorgesehen ist
dadurch gekennzeichnet,
daß die Bündelungseinrichtung ein Halteband (249) mit etwa der Breite des Flachkabels (213) umfaßt, das aus einem im wesentlichen nichtelastisch biegbaren Material besteht, im Bereich sowohl der Außentrommeldurchführöffnung (19) als auch im Bereich der Innentrommeldurchführöffnung (21) festgelegt ist und mit einer Vielzahl von über seine Länge und Breite verteilten Löchern (55) vorgesehen ist, durch welche hindurch die einzelnen Leiter (25) in das Halteband (249) in Längsrichtung eingeflochten sind.

37. Wickelvorrichtung nach Anspruch 36,
dadurch gekennzeichnet,
daß das Halteband (249) einen Endes an einer Außentrommeldurchführöffnungsklemmvorrichtung (257) und anderen Endes in Nachbarschaft der Innentrommeldurchführöffnung (21) mittels einer Halteleiste (263) an der Innentrommel (17) festgelegt ist und daß die Innentrommeldurchführöffnung (21) um die Leiter (25) herum mit einer festen Vergußmasse ausgegossen ist, welche die Leiter (25) nichtklebend umgibt, so daß sie in ihrer Längsrichtung verschiebbar sind und von der Vergußmasse nur in Position gehalten werden.

38. Wickelvorrichtung nach mindestens einem der Ansprüche 1 bis 37, in Verbindung mit einer gekrümmten Ablagebahn für den von der Außentrommel (15) abgewickelten Teil der Leitungsanordnung,
dadurch gekennzeichnet,
daß die Wickelvorrichtung (11) so über der Ablagebahn montiert ist, daß der von der Außentrommel (15) abgewickelte Teil des Außenbereichs (43) der Leitungsanordnung die gleiche Krümmungsrichtung wie die Ablagebahn aufweist.

## Claims

1. A winding device (11) for winding and unwinding a line assembly (13) having one end coupled to a stationary means and another end coupled to a reciprocable means, comprising
a stationary hollow cylindrical inner drum (17) and a hollow cylindrical outer drum (15) rotatable about the latter,
the inner drum (17) and the outer drum (15) being each provided with a line passage opening (19, 21) for passage and fixation of the line assembly (13) thereat,
in which, by rotation of the outer drum (15), an outer portion of the line assembly (13), which is located outside of the outer drum (15), is adapted to be wound on and unwound from the outer circumference (27) of the outer drum, respectively, and an inner portion of the line assembly (13), which is located between outer drum (15) and inner drum (17), is adapted to be wound and unwound, respectively, with respect to the inner drum (17),
**characterized** in
that a drive means (51, 43) driven by movement of the reciprocable means is coupled to the outer drum (15) via a sliding coupling (37, 43) and is dimensioned in relation to the outer drum diameter such that the drive means (51, 43), in case of a non-sliding coupling, would bring the outer drum (15) to a circumferential speed that would be higher than the circumferential speed of the outer drum (15) required for a winding operation of the outer portion of the line assembly (13),
and in that the outer drum (15) is subject to the braking effect of a braking means (39) whose braking force is dimensioned such that it tends to decelerate the outer drum (15) in unwinding rotational direction to a circumferential speed that is lower than the circumferential speed of the outer drum (15) corresponding to the unwinding speed of the outer portion of the line assembly (13).

2. A winding device according to claim 1,
characterized in that the drive means is constituted by a driving wheel means (51, 43) which cooperates with a rolling path (75) located beside the winding and unwinding path of the outer portion of the line assembly (13), and in that the outer drum (15) is held freely rotatable with respect to the rolling path (75).

3. A winding device according to claim 2,
characterized in that, at least on one outer face side of the outer drum (15), there is provided an outer drum disc (37) which is coaxial with the outer drum (15) and rotates together therewith and on whose outer circumference is mounted a driving wheel ring (43) which is rotatable relative to the outer drum disc (37) while overcoming a frictional force between outer drum disc (37) and driving wheel ring (43), whose outer diameter is smaller than the outer diameter of the outer drum (15) and whose outer circumference, with respect to the rolling path (75), has a high coefficient of friction preventing rolling slip.

4. A winding device according to claim 3,
characterized in that the outer circumference of the driving wheel ring (43) has a running ring (51) disposed thereon which, both with respect to the material of the rolling path (75) and with respect to the material of the driving wheel ring (43), has a high coefficient of friction preventing slip of the running ring (51).

5. A winding device according to claim 4,
characterized in that both the running ring (51) and the driving wheel ring (43) are made of elastic material, that the driving wheel ring (43) has at least one radial gap (45), and in that the driving wheel ring (43) is urged by the elastic running ring (51) onto the outer circumference of the outer drum disc (37) with a predetermined enclosing force corresponding to the necessary sliding coupling force.

6. A winding device according to at least one of claims 2 to 5,
characterized in that the outer drum disc (37) has a notched or bulging outer circumference profile and the driving wheel ring (43) has a bulging or notched inner circumference profile complementary thereto.

7. A winding device according to claim 6,
characterized in that the outer drum disc (37) has a concave or convex outer circumferential surface and the driving wheel ring has a convex or concave inner circumferential surface complementary thereto.

8. A winding device according to claim 6,
characterized in that the outer drum disc (37) has a V-shaped outer circumferential surface and the driving wheel ring (43) has a V-shaped inner circumferential surface complementary thereto.

9. A winding device according to at least one of claims 1 to 7,
characterized in that the outer drum disc is designed as outer drum ring (37) whose inner circumference revolves, with a frictional braking effect, on the outer circumference (61) of a stationary brake disc (59) coaxial therewith.

10. A winding device according to claim 9,
characterized in that the brake disc (39) has at least one radial brake disc gap (67), and that the portions of the brake disc (59) adjacent the brake disc gap (67) are biased by means of a compression spring (71) into a direction of expansion of the brake disc gap (67).

11. A winding device according to claim 9,
characterized in that the pressure force of the compression spring (71) is adjustable by means of an adjusting screw (73).

12. A winding device according to claim 10 or 11,
characterized in that the brake disc (59) is held in stationary manner only in the portion located in front of the brake disc gap (67) as seen in unwinding rotational direction of the outer drum (15), so that the portion of the brake disc (59) located behind the brake disc gap (67) as seen in this unwinding rotational direction is urged away from the stationary portion of the brake disc (59) with the assistance of the compression spring (71) and expanding the brake disc gap (67), due to the friction between outer drum ring (37) and brake disc (59) when the outer drum (15) is rotated in unwinding direction, so that the braking force of the brake disc (59) is higher when the outer drum (15) is rotated in unwinding direction than when the outer drum (15) is rotated in winding direction.

13. A winding device according to at least one of claims 9 to 12,
characterized in that the brake disc (59) is designed as a brake ring having its inner circumference disposed on the outer circumference of the inner drum (17).

14. A winding device according to at least one of claims 1 to 12,
characterized in that the inner drum (17) is disposed excentrically with respect to the axis of the outer drum.

15. A winding device according to claim 14,
characterized in that the smallest radial distance between inner drum (17) and outer drum (15) is approx. three times the line assembly thickness.

16. A winding device according to claim 14 or 15,
characterized in that the brake ring (59) is provided with a radial brake disc gap (67) at its point of largest radial ring thickness.

17. A winding device according to at least one of claims 1 to 16,
characterized in that the enveloping surface of the inner drum (17) and/or the outer drum (15) is of spiral cross-section at least in the portion of the line passage openings (19, 21), and in that the end of the spiral shape confining one side of the line passage opening (19, 21) is spaced from the end of the spiral shape confining the other side of the line passage opening (19, 21) by a radial distance corresponding approx. to the thickness of the line assembly (13).

18. A winding device according to at least one of claims 1 to 17,
characterized in that the line assembly (13) is an electrical line assembly.

19. A winding device according to claim 18,
characterized in that the line assembly (13) comprises a plurality of electrical conductors (25).

20. A winding device according to at least one of claims 1 to 19,
characterized in that the line assembly is constituted by a flat cable (13).

21. A winding device according to at least one of claims 1 to 20,
characterized in that the inner drum (17) is open on at least one face side and in that the part of the line assembly (13) adapted to be coupled to the stationary means is axially passed out of the inner cavity (23) of the inner drum (17) through the open face side.

22. A winding device according to claim 21,
characterized in that both face sides of the inner drum (17) are open and in that, of the part of the line assembly (13) to be coupled to the stationary means, a first number of conductors (25) is axially passed out through one open face side of the inner drum (17) and the remaining number of conductors (25) is axially passed out through the other open face side of the inner drum (17).

23. A winding device according to at least one of claims 1 to 22,
characterized in that the inner cavity (23) of the inner drum (17) along with the stationary part of the line asssembly (13) disposed therein is filled with cast fixing material.

24. A winding device according to at least one of claims 9 to 23,
characterized in that the inner circumference of the outer drum disc (37) and the outer circumference of the brake disc (59) have a circumferential profile according to any one of claims 6 to 8.

25. A winding device according to at least one of claims 3 to 24,
characterized in that one driving wheel ring (43) each is disposed on both face sides of the outer drum (15), that a rail (75, 77) having the cross-sectional profile of approx. a lying, upwardly open "C" extends along the winding and unwinding path of the outer portion of the line assembly (13),
that the driving wheel rings (43) and the running rings (51), respectively, perform a rolling motion on the upstanding legs (75) of the "C"-shape,
and in that the outer drum (15) is arranged between the upstanding legs (75) so as to be freely rotatable with respect to the rail (75, 77).

26. A winding device according to at least one of claims 3 to 25,
characterized in that a positive engagement exists between driving wheel ring (34) and rolling path (75).

27. A winding device according to at least one of claims 2 to 26,
characterized in that the rolling path (75) is part of a supporting member (77) for the reciprocable means.

28. A winding device according to claim 27,
characterized in that the supporting member (77) is constituted by an arcuate rail having at least on its one end a means that is reciprocable along an arcuate path by an arcuate pivoting motion of the arcuate rail.

29. A winding device according to claim 28,
characterized in that the means is an x-radiation source.

30. A winding device according to at least one of claims 3 to 29,
characterized in that the outer drum disc (37) and the driving wheel ring (43) consist of materials of different hardness at least in the region of their cooperating circumferential surfaces.

31. A winding device according to at least one of lcaims 3 to 30,
characterized in that the outer drum ring (37) and the brake disc (59) consist of materials of different hardness at least in the region of their cooperating circumferential surfaces.

32. A winding device according to claim 30 or 31,
characterized in that the outer drum ring (37) and the driving wheel ring (43) and/or the brake disc (59) consist of low pressure polyethylene (HD-PE) of different hardness at least in the region of their cooperating circumferential surfaces.

33. A winding device according to at least one of claims 1 to 32,
characterized in that a free-running means (143, 145, 151, 153, 155) having a free-running effect only in the unwinding rotational direction of the outer portion of the line assembly is provided between drive means (51, 43) and outer drum (15).

34. A winding device according to claim 33,
characterized in that the free-running means (143, 145, 151, 153, 155) is formed by a subdivision of the driving wheel ring (43) into two concentrical partial rings (143, 145) having disposed, between their opposing circumferential ring surfaces (147, 149), inhibiting rollers (151) which are movable on a radially sloping inclined surface recess (153) in one circumferential ring surface (147) of one partial ring (145) and which are of such diameter that, when positioned at the lowest point of the inclined surface recess (153), they do not project beyond this circumferential ring surface (147, but in the course of moving upwardly on the inclined surface of the inclined surface recess (153), project beyond this circumferential ring surface (147) and thereby inhibit free rotatability of the two partial rings (143, 145) with respect to each other, with the inhibiting rollers (151) being subject to spring bias (155) tending to urge them out of the lowest position of the inclined surface recess (153).

35. A winding device according to at least one of claims 1 to 34,
the line assembly having a multiplicity of flexible lines (25) commonly embedded in an outer jacket (253) of flexible material,
characterized in
- that only the outer portion (243) of the line assembly is provided with the outer jacket (253),
- that the lines (25) in the inner portion (245) of the line assembly are loosely held by a bundling means (249) approx. in that bundled shape which they have in their outer portion (243) due to being embedded in the outer jacket (253), the individual lines (25) being slidable with respect to each other and with respect to the bundling means (249),
- and in that the line assembly is passed through the line passage openings (19, 21) such that the individual lines (25) are longitudinally slidable in the region of the outer drum passage opening (19) with respect to the outer jacket (253) and in the region of the inner drum passage opening (21) with respect to said opening.

36. A winding device according to claim 35,
in which a flat cable (213) having a series of juxtaposed electrical electrical conductors (25) is provided,
characterized in that the bundling means comprises a holding band (249) which has approx. the width of the flat cable (213) and consists of a substantially non-elastically flexible material, is fixed both in the region of the outer drum passage opening (19) and in the region of the inner drum passage opening (21) and is provided with a multiplicity of holes (55) distributed across its length and width, through which the individual conductors (25) are longitudinally braided into the holding band (249).

37. A winding device according to claim 36,
characterized in that the holding band (249) is fixed at one end to an outer drum passage opening clamping device (257) and at the other end is fixed by means of a holding strip (263) to the inner drum (17) in the vicinity of the an inner drum passage opening (21), and in that the inner drum passage opening (21) is filled around the conductors (25) with a solid casting compound surrounding the conductors (25) in non-adhesive manner such that these are slidable in longitudinal direction and are only held in position by the casting compound.

38. A winding device according to at least one of claims 1 to 37, in conjunction with a curved deposition path for the part of the line assembly unwound from the outer drum (15),
characterized in the winding device (11) is mounted above the deposition path such that the part of the outer portion (43) of the line assembly unwound from the outer drum (15) has the same direction of curvature as the deposition path.

## Revendications

1. Dispositif d'enroulement (11) pour l'enroulement et le déroulement d'un arrangement de câble (13) qui est accouplé à une extrémité avec un dispositif fixe et à l'autre extrémité avec un dispositif mobile en va et vient,
comportant un tambour intérieur (17) cylindrique creux stationnaire et un tambour extérieur (15) cylindrique creux rotatif autour de celui-ci,
le tambour intérieur (17) et le tambour extérieur (15) étant chacun pourvu d'une ouverture de passage de câble (19, 21) permettant de faire passer et d'y fixer l'arrangement de câble (13),
et une zone extérieure de l'arrangement de câble (13) située en dehors du tambour extérieur (15) pouvant être enroulée et déroulée sur sa circonférence extérieure (27), en tournant le tambour extérieur (15), et une zone intérieure de l'arrangement de câble, située entre le tambour extérieur (15) et le tambour intérieur (17) pouvant être déroulée et enroulée par rapport au tambour intérieur (17), en tournant le tambour extérieur (15),
caractérisé en ce que
un dispositif d'entraînement (51, 43) entraîné par le dispositif mobile en va-et-vient est accouplé avec le tambour extérieur (15) par un accouplement à glissement (37, 43) et est dimensionné de telle manière par rapport au diamètre du tambour extérieur que le dispositif d'entraînement (51, 43), amènerait, en cas d'accouplement non glissant, le tambour extérieur (15) à une vitesse circonférentielle qui serait supérieure à la vitesse circonférentielle du tambour extérieur (15) nécessaire pour enrouler la zone extérieure de l'arrangement de câble (13),
et en ce que le tambour extérieur (15) est soumis à l'action de freinage d'un dispositif de freinage (39) dont la force de freinage est dimensionnée de telle manière qu'elle tente de freiner le tambour extérieur (15) dans le sens du déroulement à une vitesse circonférentielle qui est inférieure à la vitesse circonférentielle du tambour extérieur (15) correspondant à la vitesse de déroulement de la zone extérieure de l'arrangement de câble (13).

2. Dispositif d'enroulement selon la revendication 1, caractérisé en ce que le dispositif d'entraînement est formé par un organe à roue motrice (51, 43) qui coopère avec une voie de roulement (75) située à côté de la voie d'enroulement et de déroulement de la zone extérieure de l'arrangement de câble (13), et en ce que le tambour extérieur (15) est maintenu en rotation libre par rapport à la voie de roulement (75).

3. Dispositif d'enroulement selon la revendication 2, caractérisé en ce qu'il est prévu sur au moins une face extérieure du tambour extérieur (15) un disque de tambour extérieur (37) coaxial tournant avec celui-ci, sur la circonférence extérieure duquel est montée une bague de roue motrice (43) qui peut être tournée par rapport au disque de tambour extérieur (37) en surmontant une force de friction entre le disque de tambour extérieur (37) et la bague de roue motrice (43), bague dont le diamètre extérieur est plus petit que le diamètre extérieur du tambour extérieur (15) et dont la circonférence extérieure présente par rapport à la voie de roulement (75) un coefficient de friction élevé qui empêche un patinage de roulement.

4. Dispositif d'enroulement selon la revendication 3, caractérisé en ce qu'une bague de roulement (51) est disposée sur la circonférence extérieure de la bague de roue motrice (43), ladite bague (51) présentant tant vis-à-vis du matériau de la voie de roulement (75) que vis-à-vis du matériau de la bague de roue motrice (43) un coefficient de friction élevé qui empêche un patinage de la bague de roulement (51).

5. Dispositif d'enroulement selon la revendication 4, caractérisé en ce que tant la bague de roulement (51) que la bague de roue motrice (43) sont en matériau élastique, que la bague de roue motrice (43) présente au moins une fente radiale (45) et que la bague de roue motrice (43) est pressée par la bague de roulement (51) sur la circonférence extérieure du disque de tambour extérieur (37) avec une force de fermeture prédéterminée correspondant à la force d'accouplement glissant nécessaire.

6. Dispositif d'enroulement selon l'une au moins des revendications 2 à 5, caractérisé en ce que le disque de tambour extérieur (37) présente un profil circonférentiel extérieur entaillé ou bombé et en ce que la bague de roue motrice (43) présente le profil circonférentiel intérieur complémentaire bombé ou entaillé.

7. Dispositif d'enroulement selon la revendication 6, caractérisé en ce que le disque de tambour extérieur (37) présente une surface circonférentielle extérieure concave ou convexe et la bague de roue motrice une surface circonférentielle intérieure complémentaire convexe ou concave.

8. Dispositif d'enroulement selon la revendication 6, caractérisé en ce que le disque de tambour extérieur (37) présente une surface circonférentielle extérieure en forme de V et la bague de roue motrice (43) une surface circonférentielle intérieure en forme V complémentaire à celle-ci.

9. Dispositif d'enroulement selon l'une au moins des revendications 1 à 7, caractérisé en ce que le disque de tambour extérieur est formé comme bague de tambour extérieur (37) dont la circonférence intérieure roule sous l'effet de freinage par friction, sur la circonférence extérieure (61) d'un disque de freinage (59) coaxial stationnaire.

10. Dispositif d'enroulement selon la revendication 9, caractérisé en ce que le disque de freinage (39) présente au moins une fente de disque de freinage (67) radiale et en ce que les régions du disque de freinage (59) voisines de la fente de disque de freinage (67) sont mises sous tension au moyen d'un ressort de pression (71) dans la direction qui élargit la fente de disque de freinage.

11. Dispositif d'enroulement selon la revendication 9, caractérisé en ce que la force de pression du ressort de pression (71) est réglable au moyen d'une vis de réglage (73).

12. Dispositif d'enroulement selon la revendication 10 ou 11, caractérisé en ce que le disque de freinage (59) n'est maintenu stationnaire que dans la région située devant la fente de disque de freinage (67), vu en direction de déroulement du tambour extérieur (15), de telle sorte que la région du disque de freinage (59) qui se situe derrière la fente du disque de freinage (67), vu dans cette direction de déroulement, est repoussée de la région du disque de freinage (59) maintenue stationnaire, à la suite de la friction entre la bague de tambour extérieure (37) et le disque de freinage (59) lorsque le tambour extérieur (15) tourne en direction de déroulement, en élargissant la fente de disque de freinage (67) et grâce à l'assistance du ressort de pression (71), de telle sorte que lorsque le tambour extérieur (15) est tourné en direction de déroulement, la force de freinage du disque de freinage (59) est plus grande que lorsque le tambour extérieur (15) est tourné en direction d'enroulement.

13. Dispositif d'enroulement selon l'une au moins des revendications 9 à 12, caractérisé en ce que le disque de freinage (59) est réalisé comme couronne de freinage, dont la circonférence intérieure est agencée sur la circonférence extérieure du tambour intérieur (17).

14. Dispositif d'enroulement selon l'une au moins des revendications 1 à 12, caractérisé en ce que le tambour intérieur (17) est situé de manière excentrique par rapport à l'axe du tambour extérieur.

15. Dispositif d'enroulement selon la revendication 15, caractérisé en ce que le plus petit espace radial entre le tambour intérieur (17) et le tambour extérieur (15) est approximativement le triple de l'épaisseur de l'arrangement de câble.

16. Dispositif d'enroulement selon la revendication 14 ou 15 caractérisé en ce que la couronne de freinage (59) est pourvue d'une fente de disque de freinage (67) radiale à l'endroit où l'épaisseur radiale de la couronne est la plus grande.

17. Dispositif d'enroulement selon l'une au moins des revendications 1 à 16, caractérisé en ce que la surface de l'enveloppe du tambour intérieur (17) et/ou du tambour extérieur (15) est réalisée avec une section hélicoïdale au moins dans la région des ouvertures de passage de câble (19, 21) et en ce que l'extrémité de l'hélice délimitant un des côtés de l'ouverture de passage de câble (19, 21) présente, vis-à-vis de l'extrémité de l'hélice délimitant l'autre côté de l'ouverture de passage de câble (19, 21), un écartement radial dont l'épaisseur est approximativement celle de l'arrangement de câble (13).

18. Dispositif d'enroulement selon l'une au moins des revendications 1 à 17, caractérisé en ce que l'arrangement de câble (13) est un arrangement de câble électrique.

19. Dispositif d'enroulement selon la revendication 18, caractérisé en ce que l'arrangement de câble (13) présente une multitude de conducteurs électriques (25).

20. Dispositif d'enroulement selon l'une au moins des revendications 1 à 19, caractérisé en ce que l'arrangement de câble est formé par un câble plat (13).

21. Dispositif d'enroulement selon l'une au moins des revendications 1 à 20, caractérisé en ce que le tambour intérieur (17) est ouvert au moins sur sa face et en ce que la partie de l'arrangement de câble (13) qui peut être accouplée au dispositif stationnaire est sortie axialement de la cavité intérieure (23) du tambour intérieur (17), par la face ouverte.

22. Dispositif d'enroulement selon la revendication 21 caractérisé en ce que les deux faces du tambour intérieur (17) sont ouvertes et en ce que depuis la partie de l'arrangement de câble (13) accouplée au dispositif stationnaire, un premier nombre de conducteurs (25) est passé axialement à travers l'une des faces ouvertes du tambour intérieur (17), et le reste des conducteurs (25), à travers l'autre.

23. Dispositif d'enroulement selon l'une au moins des revendications 1 à 22, caractérisé en ce que la cavité intérieure (23) du tambour intérieur (17) contenant la partie fixe de l'arrangement de câble (13) est garnie d'un matériau de fixation.

24. Dispositif d'enroulement selon l'une au moins des revendications 9 à 23, caractérisé en ce que la circonférence intérieure du disque de tambour extérieur (37) et la circonférence extérieure du disque de freinage (59) présentent un profil circonférentiel correspondant à l'une des revendications 6 à 8.

25. Dispositif d'enroulement selon l'une au moins des revendications 3 à 24, caractérisé en ce qu'une bague de roue motrice (43) est agencée sur chacune des deux faces du tambour extérieur (15),
en ce que le long de la voie d'enroulement et de déroulement de la zone extérieure de l'arrangement de câble (13) court un rail (75, 77) dont le profil transversal a approximativement la forme d'un C couché, ouvert vers le haut,
en ce que les bagues de roue motrice (43) et les bagues de roulement (51) respectivement, roulent sur les bras verticaux du C,
et en ce que le tambour extérieur (15) est agencé entre les bras verticaux (75) en rotation libre par rapport au rail (75, 77).

26. Dispositif d'enroulement selon l'une au moins des revendications 3 à 25, caractérisé en ce qu'un blocage par coopération de forme relie la bague de roue motrice (34) et la voie de roulement (75).

27. Dispositif d'enroulement selon l'une au moins des revendications 2 à 26, caractérisé en ce que la voie de roulement (75) est une partie d'un élément de support (77) pour le dispositif mobile en va-et-vient.

28. Dispositif d'enroulement selon la revendication 27, caractérisé en ce que l'élément de support (77) est formé par un rail en forme d'arc circulaire, à au moins une extrémité duquel est agencé un dispositif qui peut être déplacé en va-et-vient par pivotement en arc de cercle du rail en forme de cercle le long d'un arc de cercle.

29. Dispositif d'enroulement selon la revendication 28, caractérisé en ce que le dispositif est une source de rayons X.

30. Dispositif d'enroulement selon l'une au moins des revendications 3 à 29, caractérisé en ce que le disque de tambour extérieur (37) et la bague de roue motrice (43) sont en des matériaux de duretés différentes, au moins dans la région de leurs surfaces circonférentielles coopérantes.

31. Dispositif d'enroulement selon l'une au moins des revendications 3 à 30, caractérisé en ce que la bague de tambour extérieur (37) et le disque de freinage (59) sont en des matériaux de duretés différentes, au moins dans la région de leurs surfaces circonférentielles coopérantes.

32. Dispositif d'enroulement selon les revendications 30 ou 31, caractérisé en ce que la bague de tambour extérieur (37) et la bague de roue motrice (43) et/ou le disque de freinage (59) sont en polyéthylène basse pression (HD-PE) de duretés différentes.

33. Dispositif d'enroulement selon l'une au moins des revendications 3 à 29, caractérisé en ce qu'il est prévu entre le dispositif d'entraînement (51, 43) et le tambour extérieur (15) un dispositif à roue libre qui n'est libre que dans le sens de déroulement de la zone extérieure de l'arrangement de câble (143, 145, 151, 153, 155).

34. Dispositif d'enroulement selon la revendication 33, caractérisé en ce que le dispositif à roue libre (143, 145, 151, 153, 155) est réalisé par division de la bague de roue motrice (43) en deux bagues partielles (143, 145) concentriques entre les surfaces annulaires circonférentielles opposées desquelles sont agencés des galets de ralentissement (151) qui sont mobiles sur une cuvette à parois obliques (153), inclinée en direction radiale dans la surface annulaire circonférentielle (147) d'une des bagues partielles (145) et présentent un tel diamètre qu'ils ne dépassent pas cette surface annulaire circonférentielle (147) lorsqu'ils sont positionnées au plus profond de la cuvette à parois obliques (153), mais qu'ils dépassent toutefois cette surface annulaire circonférentielle (147) au cours de leur montée sur les parois obliques de la cuvette (153) et empêchent ainsi que les deux bagues partielles (143, 145) puissent tourner librement l'une par rapport à l'autre, les galets de ralentissement (151) étant soumis à une prétension par ressort (155) qui tente de les faire ressortir du point le plus profond de la cuvette (153).

35. Dispositif d'enroulement selon l'une au moins des revendications 1 à 34, l'arrangement de câble présentant une multitude de conducteurs (25) flexibles qui sont enrobés ensemble dans une enveloppe extérieure (253) en matériau flexible, caractérisé en ce que
- seule la zone extérieure (243) de l'arrangement de câble est pourvue de l'enveloppe extérieure (253),
- dans la zone intérieure (245) de l'arrangement de câble, les conducteurs (25) sont maintenus de façon lâche au moyen d'un dispositif de rassemblement, approximativement dans la forme de faisceau qu'ils ont dans la zone extérieure (243) en raison de leur enrobage dans l'enveloppe extérieure (253), les câbles individuels pouvant être déplacés les uns contre les autres et par rapport au dispositif de rassemblement (249),
- l'arrangement de câble est conduit de telle manière à travers les ouvertures de passage de câble (19, 21) que les conducteurs individuels (25) peuvent être déplacés dans la région de l'ouverture de passage du tambour extérieur (19) par rapport à l'enveloppe extérieure (253) et dans la région de l'ouverture de passage du tambour intérieur (21) par rapport à celle-ci dans sa direction longitudinale.

36. Dispositif d'enroulement selon la revendication 35, un câble plat (213) étant prévu avec une rangée de conducteurs électriques (25) agencés les uns à côté des autres, caractérisé en ce que le dispositif de rassemblement comprend un ruban de support (249) qui a approximativement la largeur du câble plat (213), qui est constitué en un matériau souple essentiellement non élastique, qui est fixé tant dans la région de l'ouverture de passage du tambour extérieur (19) que dans la région de l'ouverture de passage du tambour intérieur (21) et qui est pourvu d'une multitude de trous (55) répartis sur sa longueur et sa largeur, au travers desquels les conducteurs individuels (25) sont tressés en direction longitudinale dans le ruban de support (249).

37. Dispositif d'enroulement selon la revendication 36, caractérisé en ce que le ruban de support (249) est fixé à une extrémité sur un dispositif de serrage de l'ouverture de passage du tambour extérieur (257), et à l'autre extrémité au voisinage de l'ouverture de passage du tambour intérieur (21) au moyen d'une baguette de retenue (263) sur le tambour intérieur (17) et en ce que l'ouverture de passage du tambour intérieur (21) est enrobée autour du conducteur avec une masse de scellement solide qui entoure les conducteurs (25) sans les coller, de sorte qu'ils puissent être déplacés dans leur direction longitudinale et ne soient retenus par la masse de scellement que dans leur position.

38. Dispositif d'enroulement selon l'une au moins des revendications 1 à 37, en liaison avec une voie de dépôt incurvée pour la partie de l'arrangement de câble qui est déroulée depuis le tambour extérieur (15), caractérisé en ce que le dispositif d'enroulement (11) est monté de telle manière sur la voie de dépôt que la partie de la zone extérieure (43) de l'arrangement de câble qui est déroulée depuis le tambour extérieur (15) présente la même direction de courbure que la voie de dépôt.
